# EUROPEAN PATENT APPLICATION

(11) **EP 3 683 234 A1**
(43) Date of publication of application: **22.07.2020**
(21) Application number: 18855927.2
(22) Date of filing: 12.09.2018
(51) Int. Cl.: C07K 16/24, A61K 39/00, C12N 15/13, C12N 15/63, C12N 15/85, A61P 37/00

(54) **IL-6R ANTIBODY AND ANTIGEN BINDING FRAGMENT THEREOF AND MEDICAL USE**

(30) Priority: 13.09.2017 CN 201710821975
(71) Applicant: Jiangsu Hengrui Medicine Co., Ltd., Jiangsu 222047 (CN); Shanghai Hengrui Pharmaceutical Co., Ltd., Shanghai 200245 (CN)
(72) Inventor: YING, Hua, Shanghai 200245 (CN); JIN, Houcong, Shanghai 200245 (CN); HU, Qiyue, Shanghai 200245 (CN); GE, Hu, Shanghai 200245 (CN); WANG, Yifang, Shanghai 200245 (CN); TAO, Weikang, Shanghai 200245 (CN)
(74) Representative: Danner, Stefan
(86) International application number: PCT/CN2018/105180
(87) International publication number: WO 2019/052457

(57) **Abstract**

Provided in the present invention is an IL-6R antibody or antigen-binding fragment thereof. The IL-6R antibody is an alpaca-derived antibody, chimeric antibody and humanized antibody. Also provided in the present invention is the use of the antibody in the preparation of a drug for treating IL-6-related diseases.

## Description

### FIELD OF THE INVENTION

The present disclosure relates to an IL-6R antibody or antigen-binding fragment thereof. The present disclosure also relates to chimeric antibodies and humanized antibodies comprising the CDR regions of the IL-6R antibody, to a pharmaceutical composition comprising the IL-6R antibody or antigen-binding fragment thereof, and its use as a diagnostic and therapeutic agent for diseases associated with IL-6R or IL-6.

### BACKGROUND OF THE INVENTION

Interleukin-6 (IL-6) is a pleiotropic inflammatory cytokine which regulates growth and differentiation of cells and plays an important role in mediating inflammatory response and immune response. The production of interleukin-6 and its receptor (IL-6R) is associated with the pathogenesis of various diseases such as multiple myeloma, autoimmune diseases and prostate cancer.

IL-6R is the first discovered member of the hematopoietic cytokine receptor superfamily, also known as CD126, including IL-6Rα and IL-6Rβ, i.e., signal transduction protein gp130 shared by the IL-6 family members. IL-6 binds to IL-6Rα to form an IL-6/IL-6Rα complex, which in turn binds to gp130 to form a high affinity complex (Journal of Immunology, 2006, 25(5): 475-479).

IL-6Rα is mainly expressed on the surface of hepatocytes, neutrophils, macrophages and some lymphocytes; while gp130 is expressed on the surface of all cells (Rheumatology (Oxford, England), 2010, 49(1): 15-24). In addition, there is a soluble form of IL-6R (i.e., sIL-6R). sIL-6R is transported in body fluids, and thus increases the cell type which responds to IL-6. For example, endothelial cells and synovial cells express gp130 but do not express IL-6R. Only in the presence of sIL-6R can these cells respond to IL-6 (Rheumatology (Oxford, England), 2010, 49(1): 15-24). In addition, there is a soluble fragment (sgp130) consisting of the extracellular domain of gp130, which is capable of binding to IL-6 and sIL-6R complex and has an antagonistic effect against the biological activity of IL-6.

Studies have shown that IL-6R is highly expressed in a variety of diseases, such as multiple myeloma, liver cancer and almost all types of myeloid leukemia (Journal of Experimental Hematology 2001: 9 (2) 184-187). As the target of immunotherapy, the first IL-6R monoclonal antibody in the world, Tocilizumab, was approved for marketing in 2009. The second IL-6R monoclonal antibody, Sarilumab, was also first approved for marketing in Canada in January 2017. The IL-6R antigen has been disclosed for a long time. Currently, antibodies against IL-6R have been reported, e.g., in WO1996011020A1, WO2009052454, WO2016089886, WO2005061000, US8753634, US20100215664, US7582298, US5795965, US8034344, WO2016062766, and CN101454345B. Most patents are related to indications and applications of IL-6R antibodies, such as liver cancer, central nervous system (CNS) inflammation, chronic rheumatoid arthritis and vasculitis.

However, there is still a need for improvement of the above-mentioned drugs in the treatment of IL-6-mediated diseases and the like. Therefore, it needs to continue to develop antibodies with high selectivity, good efficacy and low side effects, and provide more and better therapeutic regimen with anti-IL-6R for the treatment of diseases associated with IL-6R.

### SUMMARY OF THE INVENTION

The present disclosure provides a monoclonal antibody or antigen-binding fragment thereof which binds to the amino acid sequence or the three-dimensional structure of IL-6R extracellular region. Furthermore, the present disclosure provides anti-human IL-6R antibodies with high activity and high stability, which compete with said monoclonal antibody or antibody fragment thereof.

Further, the present disclosure provides a method for producing clone of the antibody according to the present disclosure, DNA encoding the antibody, a vector comprising the DNA, and a transformant obtained by transforming the vector. The present disclosure also provides a method of producing the antibody or antibody fragment thereof with the clone or the transformant; and the present disclosure also provides a diagnostic or therapeutic agent comprising the antibody or antibody fragment thereof according to the present disclosure as an active ingredient.

In one aspect, the disclosure provides a monoclonal antibody or antigen-binding fragment thereof. The monoclonal antibody or antigen-binding fragment thereof according to the present disclosure comprises CDR1, CDR2 and CDR3, wherein the sequence of CDR1 is selected from the group consisting of SEQ ID NO: 15 and a variant sequence of SEQ ID NO: 15. In a specific embodiment, the variant sequence of SEQ ID NO: 15 has 1, 2 or 3 amino acid differences from the sequence as set forth in SEQ ID NO: 15. In a specific embodiment, the variant sequence of SEQ ID NO: 15 is obtained via antibody affinity maturation test. The sequence of CDR2 is selected from the group consisting of SEQ ID NO: 16 and a variant sequence of SEQ ID NO: 16. In a specific embodiment, the variant sequence of SEQ ID NO: 16 has 1, 2 or 3 amino acid differences from the sequence as set forth in SEQ ID NO: 16. In a specific embodiment, the variant sequence of SEQ ID NO: 16 is obtained via antibody affinity maturation test. The sequence of CDR3 is selected from the group consisting of SEQ ID NO: 17 and a variant sequence of SEQ ID NO: 17. In a specific embodiment, the variant sequence of SEQ ID NO: 17 has 1, 2 or 3 amino acid differences from the sequence as set forth in SEQ ID NO: 17. In a specific embodiment, the variant sequence of SEQ ID NO: 17 is obtained via antibody affinity maturation test. The monoclonal antibody or antigen-binding fragment thereof recognizes and binds to human IL-6R.

In some embodiments, in the monoclonal antibody or antigen-binding fragment thereof, the CDR1 sequence is set forth in SEQ ID NO: 15; the CDR2 sequence is set forth in SEQ ID NO: 16; and the CDR3 sequence is set forth in SEQ ID NO:17.

In some embodiments, the monoclonal antibody is a recombinant antibody. In some embodiments, the monoclonal antibody is selected from the group consisting of alpaca-derived antibody, chimeric antibody, humanized antibody and antigen-binding fragment thereof. In some embodiments, the FR region sequence on the variable region of the humanized antibody is derived from human germline heavy chain or mutated sequence thereof.

In some embodiments, the monoclonal antibody comprises VHH of SEQ ID NO: 14 or a variant thereof; the variant has 1 to 15 amino acid mutations in the VHH sequence as set forth in SEQ ID NO: 14. More specifically, the variant of VHH has 1 to 15 amino acid mutations in the FR region of VHH as set forth in SEQ ID NO: 14. More specifically, the variant of VHH comprises mutation(s) selected from the group consisting of A14P, E23A, Q44G, V78L, K86R, N96A, A97F, Q116L and combination thereof, in the VHH sequence as set forth in SEQ ID NO: 14.

In some embodiments, the monoclonal antibody or antigen-binding fragment thereof further comprises a human antibody Fc region; preferably comprises a human antibody Fc region as set forth in SEQ ID NO: 19.

In some embodiments, the antigen-binding fragment is selected from the group consisting of a single domain antibody and a peptide comprising the CDR1 to CDR3. In some embodiments, a monoclonal antibody or antigen-binding fragment thereof according to the present disclosure competes with the monoclonal antibody or antigen-binding fragment thereof as defined above for binding to IL-6R and blocks the binding of IL-6 to IL-6R.

In another aspect, the present disclosure provides a pharmaceutical composition comprising a therapeutically effective amount of the above monoclonal antibody or antigen-binding fragment thereof, and one or more pharmaceutically acceptable carriers, diluents or excipients.

In another aspect, the present disclosure provides a nucleic acid molecule encoding the above monoclonal antibody or antigen-binding fragment thereof.

In some embodiments, the nucleic acid molecule encodes the CDR regions described above. In some embodiments, the nucleic acid molecule comprises polynucleotide having at least 80%, at least 85%, at least 90%, at least 92%, at least 94%, at least 95%, at least 96%, at least 97%, at least 98%, at least 99% or at least 100% homology to SEQ ID NO:13.

In another aspect, the present disclosure provides a recombinant vector comprising the above nucleic acid molecule.

In another aspect, the present disclosure provides a host cell transformed with the above recombinant vector; the host cell is selected from the group consisting of prokaryotic cells and eukaryotic cells, preferably eukaryotic cells, more preferably mammalian cells or yeast cells. The mammalian cells are preferably HEK293E cells, CHO cells or NS0 cells. The mammalian cells will not develop into an individual.

In another aspect, the present disclosure provides a method for producing the above monoclonal antibody or antigen-binding fragment thereof, the method comprises the steps of: cultivating the above host cell; forming and accumulating the above monoclonal antibody or antigen-binding fragment thereof in the culture; and recovering the monoclonal antibody or antigen-binding fragment thereof from the culture.

In another aspect, the present disclosure provides a method for detecting or determining human IL-6R, the method comprises the step of detecting IL-6R in a sample by the monoclonal antibody or antigen-binding fragment thereof described above. In another aspect, the present disclosure provides an agent for detecting or determining human IL-6R, the agent comprises the above monoclonal antibody or antigen-binding fragment thereof.

In another aspect, the present disclosure provides a method for treating a disease associated with human IL-6R, the method comprises administering to a subject the monoclonal antibody or antigen-binding fragment thereof as described above, or administering to a subject a pharmaceutical composition comprising the monoclonal antibody or antigen-binding fragment thereof, or administering to a subject the nucleic acid molecule as described above, for treating a disease associated with human IL-6R.

In another aspect, the present disclosure provides the use of the above monoclonal antibody or antigen-binding fragment thereof for the manufacture of a therapeutic agent for the treatment of a disease associated with IL-6; the present disclosure also provides the use of the above pharmaceutical composition for the manufacture of a therapeutic agent for the treatment of a disease associated with IL-6; the present disclosure provides the use of the above nucleic acid molecule for the manufacture of a therapeutic agent for the treatment of a disease associated with IL-6.

Furthermore, the present disclosure includes an agent for treating a disease associated with IL-6R-positive cells, the agent comprises the monoclonal antibody or antibody fragment thereof according to the present disclosure as an active ingredient.

The antibody, antigen-binding fragment and composition of the present disclosure can be used to prevent and treat a disease and a condition associated with IL-6R, IL-6, and/or associated with IL-6/IL-6R complex (optionally a complex further bound to gp130), and/or associated with signaling pathway and/or biological functions and responses involving IL-6 and/or IL-6/IL-6R complex (optionally a complex further bound to gp130). In particular, the antibody, antigen-binding fragment and composition of the present disclosure can be used to prevent and treat a disease and a condition associated with IL-6R, IL-6, and/or associated with IL-6/IL-6R complex (optionally a complex further bound to gp130), and/or associated with signaling pathway and/or biological functions and responses involving IL-6R, IL-6 and/or IL-6/IL-6R complex (optionally a complex further bound to gp130), wherein the disease and the condition is characterized by excess and/or unwanted signaling mediated by IL-6R or by pathway involving IL-6R. According to the present disclosure, those skilled in the art will apparently understand the examples of the disease and a condition associated with IL-6R, IL-6, and/or associated with IL-6/IL-6R complex, and/or associated with signaling pathway and/or biological functions and responses involving IL-6 and/or IL-6/IL-6R complex. The disease and the condition are also commonly referred to herein as "condition associated with IL-6" or "disease associated with IL-6R".

In another aspect, the present disclosure provides use of the above monoclonal antibody or antigen-binding fragment thereof, the above pharmaceutical composition, the nucleic acid molecule as described above, or combination thereof, for preparing a medicament for preventing or treating a disease or condition selected from the group consisting of: Sepsis (Starnes et al, 1999) and various cancers, such as multiple myeloma (MM), renal cell carcinoma (RCC), plasma cell leukemia (Klein et al, 1991), lymphoma, B-lymphoid hyperplasia disease (BLPD) and prostate cancer. Non-limiting examples of other diseases caused by excess IL-6 production or signaling include bone resorption (osteoporosis) (Roodman et al, 1992; Jilka et al, 1992), cachexia (Strassman et al, 1992), psoriasis, glomerular mesangial proliferative glomerulonephritis, Kaposi's sarcoma, AIDS-related lymphoma (Emilie et al, 1994); inflammatory diseases and conditions, such as rheumatoid arthritis, systemic onset of juvenile idiopathic arthritis, hypergammaglobulinemia (Grau et al., 1990); regional enteritis, ulcerative colitis, systemic lupus erythematosus (SLE), multiple sclerosis, Castleman's disease, IgMγ-globulin disease, cardiac myxoma, asthma (especially allergic asthma), inflammatory anemia, and autoimmune insulin-dependent diabetes (Campbell et al, 1991).

Furthermore, the present disclosure relates to an immunodetection or immunoassay method of IL-6R; reagents for immunodetection or immunoassay of IL-6R; an immunodetection or immunoassay of cells expressing IL-6R; and a diagnostic agent for diagnosing a disease associated with IL-6, which comprises the monoclonal antibody or antibody fragment of the present disclosure as an active ingredient, which specifically recognizes human IL-6R and binds to the amino acid sequence of the extracellular region or the three-dimensional structure thereof. In the present disclosure, the method for detecting or determining the amount of IL-6R may be any method known in the art. For example, it includes immunodetection or immunoassay.

The immunodetection or immunoassay is a method for detecting or determining the amount of antibody or antigen by using a labeled antigen or antibody. Examples of immunodetection or immunoassay include radioactive substance labeled immunological antibody method (RIA), enzyme immunoassay (EIA or ELISA), fluorescent immunoassay (FIA), luminescent immunoassay, Western Blotting, physicochemical assays, and the like.

The above diseases associated with IL-6 can be diagnosed by detecting or determining the expressed IL-6 with the monoclonal antibody or antibody fragment of the present disclosure. In order to detect cells expressing the polypeptide, a known immunoassay can be used, preferably immunoprecipitation, fluorescent cell staining, immunohistochemical staining, and the like can be used. Further, a fluorescent antibody staining method with FMAT8100HTS system (Applied Biosystem), and the like can be used.

In the present disclosure, there is not particular limitation for the living sample used for detecting or determining IL-6Rd, as long as it is likely to contain cells expressing IL-6R, for example tissue cells, blood (whole blood, plasma or serum), pancreatic fluid, urine, feces, tissue fluid or culture medium can be used.

The diagnostic agent comprising the monoclonal antibody or antibody fragment thereof of the present disclosure may further comprise an agent for performing antigen-antibody reaction or an agent for detecting the reaction, depending on the desired diagnostic method. The agent for performing antigen-antibody reaction includes such as buffer and salts. The agent for detecting the reaction includes reagents commonly used in immunodetection or immunoassay, for example, such as a labeled secondary antibody recognizing the monoclonal antibody, the antibody fragment thereof or the conjugate thereof, as well as a substrate corresponding to the labels.

### DESCRIPTION OF THE DRAWINGS

Figures 1A to 1C display an assay of blocking the binding between IL-6 and IL-6R, by 1764 and humanized antibodies thereof.
Figures 2A to 2B display an assay of blocking the binding between gp130 and IL-6/IL-6R, by 1764 and humanized antibodies thereof.
Figure 3 displays a binding assay of 1764 and U266B1 cell.
Figure 4 displays an assay of inhibiting IL-6-induced TF-1 proliferation by 1764 antibody.

### DETAILED DESCRIPTION OF THE INVENTION

### 1. Terminology

In order to more easily understand the present disclosure, certain technical and scientific terms are specifically defined below. Unless otherwise defined explicitly herein, all other technical and scientific terms used herein have the meaning commonly understood by one of ordinary skilled in the art to which this disclosure belongs.

The three-letter code and the one-letter code for amino acids used in the present disclosure are as described in J. Biol. Chem. 243, p3558 (1968).

A conventional immunoglobulin is a tetramer composed of two heavy chains and two light chains, with a combined molecular weight of about 150 kDa. A significant proportion of serum antibodies in Camelidae (camels) members are homodimeric IgGs, with a molecular weight of approximately 80 kD (Hamers-Casterman et al. 1993 Nature, 363, 446-448). These heavy chain immunoglobulins (Igs) comprise three domains, the variable regions of which are referred to as VHHs (variable domains of heavy chain of heavy-chain antibody). Recombinant VHH (approximately 12 to 14 kD) constitutes the entire antigen binding domain and exhibits a broad antigen binding profile. Expanding their hypervariable regions exhibit unique property, for example three to four hydrophobic framework residues (which interact with conventional antibody VL) are replaced by more hydrophilic amino acids. In order to stabilize the enlarged CDRs, in addition to conventional disulfide bonds, VHH may have additional disulfide bond(s) between CDR1 and CDR3 in the case of dromedary, and between CDR2 and CDR3 in the case of llama (Harmsen and De Haard 2007 Appl Microbiol Biotechnol., 77, 13-22; Muyldermans 2001 J Biotechnol., 74, 277-302). The enlarged CDR3 loop can adopt convex conformation, while the conventional paratope is confined to a concave or planar structure (Muyldermans 2001 J Biotechnol., 74, 277-302). These features allow VHH to recognize unique epitopes which are less immunogenic when compared with conventional antibodies (Lafaye et al. 2009 Mol. Immunol., 46, 695-704; Wernery 2001 J Vet Med B Infect Dis Vet Public Health., 48, 561-568). Although VHH is defined as a monovalent antibody, any affinity effect is excluded by default, and the biological activity is measured as in vitro IC50, which is similar to that of a conventional bivalent antibody molecule (Thys et al. 2010 Antiviral Res., 87: 257-264).

The term "monoclonal antibody" refers to an antibody obtained from a population of substantially homologous antibodies, i.e., each antibody comprised in the population is identical (except for possible natural mutations that may be present in minor amount). A monoclonal antibody is highly specific for a single antigenic site. Furthermore, unlike conventional polyclonal antibody preparations, which typically include different antibodies directed against different determinants (epitopes) on the antigen, each monoclonal antibody is directed only to a single determinant or epitope on the antigen.

In certain embodiments, the disclosure may be directed to a chimeric camelidae/human antibody, particularly a chimeric antibody in which the VH and/or VL domain is entirely camelidae sequence (e.g., llama or Alpaca), while the rest of the antibody completely consists of human sequence. In some preferred embodiments of the present disclosure, it further relates to "humanized" or "germlined" camelidae antibody and a chimeric camelidae/human antibody, in which the VH and/or VL domain contains one or more amino acid substitutions in the framework region, relative to the camelidae VH and/or VL domain obtained by active immunization. The percent sequence identity relative to human germline VH or VL domain is increased by such "humanization" process, i.e., replacing the mismatched amino acid residues located in the original camelidae VH or VL domain with the corresponding residues located in the human germline VH or VL domain.

The present disclosure includes natural, recombinant VHH or VH. "Recombination" involves production of the VHH or VH with genetic engineering methods (cloning, amplification). The VHH according to the present disclosure may be in the form of a monomer or a homopolymer, such as a homodimer or a homotrimer.

About 110 amino acid sequences adjacent to the N-terminus of the antibody heavy and light chains are highly variable, known as variable region (Fv region); the rest of amino acid sequences close to the C-terminus are relatively conservative, known as constant region. The variable region includes three hypervariable regions (HVRs) and four relatively conserved framework regions (FRs). The three hypervariable regions which determine the specificity of the antibody are also known as the complementarity determining regions (CDRs). Each light chain variable region (LCVR) and each heavy chain variable region (HCVR) consists of three CDR regions and four FR regions, with sequential order from the amino terminus to carboxyl terminus in the following order: FR1, CDR1, FR2, CDR2, FR3, CDR3, and FR4. The three CDR regions of the light chain refer to LCDR1, LCDR2, and LCDR3, and the three CDR regions of the heavy chain refer to HCDR1, HCDR2, and HCDR3. The number and position of CDR amino acid residues in the LCVR and HCVR regions of the antibody or antigen binding fragments herein comply with known Kabat numbering criteria (LCDR1-3, HCDR1-3).

The antibody of the present disclosure includes alpaca-derived antibodies, chimeric antibodies, humanized antibodies, preferably humanized antibodies.

The term "alpaca-derived antibody" is an alpaca-derived monoclonal antibody against human IL-6R and may be prepared according to the knowledge and skill in the art. For preparation of such antibody, the subject is injected with an IL-6R antigen, and then a hybridoma expressing the antibody having the desired sequence or functional property is isolated, or an immunological library is established and the antibody having the desired function is isolated by phage display technology. In a specific embodiment of the present disclosure, the alpaca-derived IL-6R antibody or antigen-binding fragment thereof may further comprise alpaca heavy chain Fc region.

The term "chimeric antibody" is an antibody which is formed by fusing the variable region of an alpaca-derived antibody with the constant region (or Fc region) of a human antibody, and the chimeric antibody has alleviated immune response induced by the alpaca-derived antibody. To construct a chimeric antibody, first, a hybridoma or an antibody library that secretes a specific alpaca-derived monoclonal antibody is constructed, and then alpaca-derived variable region gene is ligated with the human constant region gene (or Fc region) to form a chimeric gene which is subsequently inserted into an expression vector, and finally a chimeric antibody molecule is expressed in the eukaryotic or prokaryotic system. In a preferred embodiment of the present disclosure, the heavy chain of the IL-6R chimeric antibody further comprises the heavy chain constant region (or Fc region) of human IgG1, IgG2, IgG3, or IgG4, or variant thereof, preferably comprises the heavy chain constant region (or Fc region) of human IgG1, IgG2, or IgG4, or a variant of IgG1, IgG2, or IgG4 heavy chain constant region (or Fc region) comprising amino acid mutations (such as YTE mutation or back-mutation)

The term "humanized antibody" refers to an antibody generated by grafting alpaca CDR sequences into a variable region framework of human antibody, namely an antibody produced from different types of human germline antibody framework sequences. A humanized antibody overcomes disadvantage of the heterologous reaction induced by the chimeric antibody, which carries a large amount of alpaca protein components. Such framework sequences can be obtained from a public DNA database covering germline antibody gene sequences or published references. For example, germline DNA sequences of human heavy and light chain variable region genes can be found in "VBase" human germline sequence database (available on web www.mrccpe.com.ac.uk/vbase), as well as can be found in Kabat, E A, et al, 1991 Sequences of Proteins of Immunological Interest, 5th Ed. To avoid a decrease in activity caused by the decreased immunogenicity, the framework sequences in the variable region of human antibody are subjected to minimal reverse mutations or back mutations to maintain the activity. The humanized antibody of the present disclosure also comprises humanized antibody on which CDR affinity maturation is performed by phage display. To avoid a decrease in activity caused by the decreased immunogenicity, on the basis of the sequences of 1764, gradual mutation toward human sequence is performed, that is, gradually replace the alpaca-derived residues located on FR region of 1764 with human-derived residues to maintain the activity.

The term "VHH" relates to variable antigen binding domain derived from a heavy chain antibody of Camelidae (camel, dromedary, llama, alpaca, etc.) (see Nguyen et al. 2000 EMBO J., 19, 921-930; Muyldermans 2001 J Biotechnol. 74, 277-302 and review Vanlandschoot et al. 2011 Antiviral Res. 92, 389-407).

Generally, a nanobody can be defined as amino acid sequences having the following (general) structure: FR1-CDR1-FR2-CDR2-FR3-CDR3-FR4, wherein FR1-FR4 refer to framework regions 1-4, and wherein CDR1-CDR3 refer to complementarity determining regions 1-3, respectively.

The term "antibody frame(work) region" as used herein, refers to a portion of the variable domain (VH) which serves as a scaffold for the antigen binding loop (CDR) of the variable domain. Essentially, it refers to a variable domain without CDR.

The term "antigen-binding fragment" or "functional fragment" of an antibody refers to one or more fragments of an antibody that retain the ability to specifically bind to an antigen (e.g., IL-6R). It has been shown that antigen binding function of an antibody can be achieved by fragments of the full antibody. Examples of the binding fragment comprised in the term "antigen-binding fragment" of an antibody include:
(i) Fab fragment, a monovalent fragment consisting of VL, VH, CL and CH1 domain;
(ii) F(ab')₂ fragment, a bivalent fragment comprising two Fab fragments linked by a disulfide bridge located on the hinge region;
(iii) Fd fragment consisting of VH and CH1 domain;
(iv) Fv fragment consisting of VH and VL domain of a single arm of the antibody;
(v) a single domain or dAb fragment (Ward et al, (1989) Nature 341:544-546), which consists of VHH domain;
(vi) an isolated complementarity determining region (CDR); or
(vii) a combination of two or more separate CDRs, optionally linked by a synthetic linker;
(viii) a fusion protein formed by VHH and Fc;
(ix) a fusion protein formed by VHH and antibody heavy chain constant region.

In addition, "antigen-binding fragment" also includes other antigen-binding forms comprising the three CDRs in VHH domain which is capable of binding to IL-6R.

The disclosure further encompasses such an antigen binding polypeptide, in which hypervariable loops or CDRs of the VHH domain are derived from camelidae, while at least one of the hypervariable loops or CDRs derived from camelidae is engineered to contain one or more amino acid substitutions, additions or deletions, relative to the coding sequence derived from camelidae. These changes include the "humanization" of hypervariable loops/CDRs. The camelidae-derived HV/CDRs engineered in this manner can still exhibit amino acid sequences "substantially identical" to those of the HV/CDRs encoded by camelidae. In this case, "substantially identical" may allow no more than one, or no more than two, or no more than three amino acid sequence mismatches when compared to the HV/CDRs encoded by camelidae.

The antibodies of the present disclosure may be of any isotype. The types of antibodies for human therapeutic use are typically of IgA, IgD, IgE, IgG, IgM type. Usually the antibodies are IgG type antibodies, in such case, they may belong to any one of the four subtypes, i.e., IgGI, IgG2a and IgG2b, IgG3 or IgG4. In these subtypes, one or more amino acid substitutions, insertions or deletions are allowed in the Fc portion, or other structural modifications are allowed to, for example, increase or decrease Fc-dependent function.

The diabody of the present disclosure can be produced by the steps of: obtaining cDNA encoding the VHH of the monoclonal antibody of the present disclosure which specifically recognizes human IL-6R and binds to the extracellular region (amino acid sequence or three-dimensional structure thereof), constructing a DNA encoding the VHH, such that the peptide linker is 8 or less amino acid residues in length, inserting the DNA into a prokaryotic expression vector or a eukaryotic expression vector, and then introducing the expression vector into a prokaryote or eukaryote to express the diabody.

A CDR-containing peptide is constructed by one or more regions of CDRs comprising VHH. Peptides comprising several CDRs can be joined directly or via a suitable peptide linker. The CDR-containing peptide of the present disclosure can be produced by: constructing a DNA encoding the CDR of VHH of the monoclonal antibody of the present disclosure which specifically recognizes human IL-6R and binds to the extracellular region (amino acid sequence or three-dimensional structure thereof), inserting the DNA into a prokaryotic or eukaryotic expression vector, and then introducing the expression vector into a prokaryote or eukaryote to express the peptide. The CDR-containing peptide can also be produced by a chemical synthesis method such as Fmoc method or tBoc method.

The term "epitope" or "antigenic determinant" refers to a site on an antigen to which an immunoglobulin or antibody specifically binds (e.g., a specific site on the IL-6R molecule). Epitopes typically include at least 3, 4, 5, 6, 7, 8, 9, 10, 11, 12, 13, 14 or 15 consecutive or non-consecutive amino acids in a unique tertiary conformation. See, for example, Epitope Mapping Protocols in Methods in Molecular Biology, Vol. 66, G. E. Morris ed. (1996).

The term "specific binding", "selective binding", "selectively binds to" and "specifically binds to" refers to the binding of an antibody to a predetermined epitope on an antigen. Typically, the antibody binds with an affinity (K_{D}) of less than about 10⁻⁷ M, for example, less than about 10⁻⁸ M, 10⁻⁹ M, or 10⁻¹⁰ M or even less.

The term "K_{D}" or "Kd" refers to the dissociation equilibrium constant for particular antibody-antigen interaction. Typically, the antibody of the present disclosure binds to IL-R6 with a dissociation equilibrium constant (K_{D}) of less than about 10⁻⁷ M, such as less than about 10⁻⁸ M, 10⁻⁹ M or 10⁻¹⁰ M or even less, for example, as determined using surface plasmon resonance (SPR) techniques in a BIACORE instrument.

When the term "competition" is used in the context of antigen binding proteins (e.g., neutralizing antigen binding proteins or neutralizing antibodies) that compete for the same epitope, it means that competition occurs between the antigen binding proteins, which is determined by the following assays: the antigen binding protein to be tested (e.g., an antibody or immunologically functional fragment thereof) prevents or inhibits (e.g., reduces) the specific binding between a reference antigen binding protein (e.g., a ligand or reference antibody) and a common antigen (e.g., an IL-6R antigen or fragment thereof). Numerous types of competitive binding assays are available to determine whether an antigen binding protein competes with another. These assays are, for example, solid phase direct or indirect radioimmunoassay (RIA), solid phase direct or indirect enzyme immunoassay (EIA), Sandwich competition assay (see, e.g., Stahli et al, 1983, Methods in Enzymology 9: 242-253); solid phase direct biotin-avidin EIA (see, e.g., Kirkland et al, 1986, J. Immunol. 137: 3614-3619), solid phase direct labeling assay, solid phase direct labeling sandwich assay (see, e.g., Harlow and Lane, 1988, Antibodies, A Laboratory Manual, Cold Spring Harbor Press); solid phase direct labeling RIA with I⁻¹²⁵ label (see, e.g., Morel et al, 1988, Molec. Immunol. 25: 7-15); solid phase direct biotin-avidin EIA (see, e.g., Cheung, et al, 1990, Virology 176: 546-552); and direct labeling RIA (Moldenhauer et al, 1990, Scand. J. Immunol. 32: 77-82). Typically, the assay involves the use of a purified antigen (either on a solid surface or on a cell surface) capable of binding to both an unlabeled antigen binding protein to be tested and a labeled reference antigen binding protein. Competitive inhibition is determined by measuring the amount of label bound to the solid surface or to the cell in the presence of the antigen binding protein to be tested. Usually, the antigen binding protein to be tested is present in excess. Antigen binding proteins identified by competitive assay (competing with the antigen binding protein) includes: antigen binding proteins that bind to the same epitope as the reference antigen binding protein; and antigen binding proteins that bind to an epitope that is sufficiently close to the epitope to which the reference antigen binding protein binds, where the two epitopes spatially interfere with each other to hinder the binding. Additional details regarding methods for determining competitive binding are provided in the Examples herein. Typically, when a competing antigen binding protein is present in excess, it will inhibit (e.g., reduce) at least 40-45%, 45-50%, 50-55%, 55-60%, 60-65%, 65-70%, 70-75% or 75% or even more of the specific binding between the reference antigen binding protein and the common antigen. In some cases, binding is inhibited by at least 80-85%, 85-90%, 90-95%, 95-97%, or 97% or even more.

The term "nucleic acid molecule," as used herein refers to DNA molecules and RNA molecules. A nucleic acid molecule may be single-stranded or double-stranded, but preferably is double-stranded DNA. A nucleic acid is deemed as "operably linked" when it is placed into a functional relationship with another nucleic acid sequence. For instance, a promoter or enhancer is operably linked to the coding sequence if it affects the transcription of the sequence.

The term "vector" refers to a nucleic acid molecule capable of transporting another nucleic acid with which it has been linked. In one embodiment, the vector is a "plasmid," which refers to a circular double stranded DNA loop into which additional DNA segments may be ligated. In another embodiment, the vector is a viral vector, wherein additional DNA segments may be ligated into the viral genome. The vectors disclosed herein are capable of self-replicating in the host cell into which they have been introduced (e.g., bacterial vectors having bacterial replication origin and episomal mammalian vectors), or may be integrated into the genome of a host cell upon introduction into the host cell, and thereby are replicated along with the host genome (e.g., non-episomal mammalian vectors).

Methods for producing and purifying antibodies and antigen-binding fragments are well known in the art and can be found, for example, in Antibodies, A Laboratory Manual, Cold Spring Harbor Laboratory Press, chapters 5-8 and 15. For example, mice can be immunized with human IL-6R or fragments thereof, and the resulting antibodies can then be renatured, purified, and sequenced for amino acid sequences by using conventional methods well known in the art. Antigen-binding fragments can also be prepared by conventional methods. The antibodies or antigen binding fragments of the present disclosure are engineered to introduce one or more human framework regions into CDRs derived from a non-human antibody. Human FR germline sequences can be obtained by aligning human antibody variable germline gene database and MOE software, and are available from ImMunoGeneTics (IMGT) website http://imgt.cines.fr, or from The Immunoglobulin Facts Book, 2001, ISBN 012441351.

The term "host cell" refers to a cell into which the expression vector has been introduced. Host cells may include microbial (e.g. bacterial), plant or animal cells. Bacteria that are susceptible to be transformed include members of *Enterobacteriaceae,* such as *Escherichia coli* or *Salmonella* strains; *Bacillaceae* such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include, but are not limited to CHO (Chinese hamster ovary cell line), HEK cells (as non-limiting examples, HEK293E cells) and NS0 cells, etc.

The engineered antibodies or antigen binding fragments of the present disclosure may be prepared and purified using known methods. For example, cDNA sequence encoding a heavy chain and a light chain may be cloned and recombined into a GS expression vector. The recombinant immunoglobulin expression vector may then be stably transfected into CHO cells. As a more recommended method well known in the art, mammalian expression systems will result in glycosylation, typically at highly conserved N-terminal sites in the Fc region. Stable clones may be verified for expression of antibody specifically binding to human IL-6R. Positive clones may be expanded in serum-free culture medium in bioreactors for antibody production. Culture medium, into which an antibody has been secreted, may be purified by conventional techniques. For example, Protein A or G Sepharose FF column that has been equilibrated with adjusting buffer can be used for purification. The column is washed to remove nonspecific binding components, and then the bound antibody is eluted by pH gradient and antibody fractions are detected, by SDS-PAGE, and then collected. The antibodies may be filtered and concentrated using common techniques. Soluble aggregates and polymers may be effectively removed by common techniques, such as size exclusion or ion exchange. The resulting product is then immediately frozen, for example at -70°C, or may be lyophilized.

"Administration", "administering" or "treatment," as it applies to an animal, human, subject, cell, tissue, organ, or biological fluid, refers to contacting an exogenous pharmaceutical, therapeutic, diagnostic agent, or composition with the animal, human, subject, cell, tissue, organ, or biological fluid. "Administration", "administering" or "treatment" can refer, e.g., to therapeutic, pharmacokinetic, diagnostic, research and experimental methods. Treatment of a cell encompasses contacting a reagent with the cell, as well as contacting a reagent with a fluid, where the fluid is in contact with the cell. "Administration", "administering" or "treatment" also means in vitro and ex vivo treatments, e.g., of a cell, with a reagent, diagnostic, binding composition, or with another cell. "Treatment," as it applies to human, veterinary, or research subject, refers to therapeutic treatment, prophylactic or preventative measures, research and diagnostic applications.

"Treat" means to administer a therapeutic agent, such as a composition containing any of antibodies or fragment thereof of the present disclosure, internally or externally to a patient having one or more disease symptoms for which the agent has known therapeutic activity. Typically, the agent is administered in an amount effective to alleviate one or more disease symptoms in the patient or population to be treated, whether by inducing the regression of or inhibiting the progression of such symptom(s) by any clinically measurable degree. The amount of a therapeutic agent that is effective to alleviate any particular disease symptom (also referred to as the "therapeutically effective amount") may vary according to factors such as the disease state, age, and weight of the patient, and the ability of the drug to elicit a desired response in the patient. Whether a disease symptom has been alleviated can be assessed by any clinical measurement typically used by physicians or other skilled healthcare providers to assess the severity or progression status of that symptom. While an embodiment of the present disclosure (e.g., a treatment method or manufacture article) may not be effective in alleviating the target disease symptom(s) in every patient, it should alleviate the target disease symptom(s) in a statistically significant number of patients as determined by any statistical test known in the art such as the Student's t-test, the chi-square test, the U-test according to Mann and Whitney, the Kruskal-Wallis test (H-test), Jonckheere-Terpstra-test and the Wilcoxon-test.

"Conservative modification" or "conservative substitution or replacement" refers to substitutions of amino acids in a protein with other amino acids having similar characteristics (e.g. charge, side-chain size, hydrophobicity/hydrophilicity, backbone conformation and rigidity, etc.), such that the changes can frequently be made without altering the biological activity of the protein. Those of skill in this art recognize that, in general, single amino acid substitution in non-essential regions of a polypeptide does not substantially alter biological activity (see, e.g., Watson et al. (1987) Molecular Biology of the Gene, The Benjamin/ Cummings Pub. Co., p. 224 (4th Ed.)). In addition, substitutions with structurally or functionally similar amino acids are less likely to disrupt biological activity.

"Effective amount" means an amount sufficient to ameliorate or prevent a symptom or sign of the medical condition. Effective amount also means an amount sufficient to allow or facilitate diagnosis. An effective amount for a particular patient or veterinary subject may vary depending on factors such as the condition being treated, the overall health condition, the route and dose of administration and the severity of side effects. An effective amount can be the maximal dose or dosing protocol that avoids significant side effects or toxic effects.

"Exogenous" refers to substances that are produced outside an organism, cell, or human body, depending on the context. "Endogenous" refers to substances that are produced within a cell, organism, or human body, depending on the context.

"Homology" refers to sequence similarity between two polynucleotide sequences or between two polypeptide sequences. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions to be compared, and then multiplied by 100. For example, when the sequences are optimally aligned, if 6 out of 10 positions in two sequences are matched or homologous then the two sequences have 60% homology; if 95 out of 100 positions in two sequences are matched or homologous, then the two sequences have 95% homology. Generally, the comparison is performed when two sequences are aligned to give maximum % homology.

As used herein, the expressions "cell," "cell line," and "cell culture" are used interchangeably and all such designations include progeny thereof. Thus, the terms "transformants" and "transformed cells" include the primary subject cells and cultures derived therefrom, regardless of the number of transfers or passages. It should be also understood that all progeny may not be precisely identical in DNA content, due to deliberate or inadvertent mutations. Mutant progeny thus screened that have the same function or biological activity as that of originally transformed cells are included. Where distinct designations are intended, it will be clearly understood from the context.

As used herein, "polymerase chain reaction" or "PCR" refers to a procedure or technique in which minute amount of a specific portion of nucleic acid, RNA and/or DNA, is amplified as described in, e.g., U.S. Pat. No. 4,683,195. Generally, sequence information at the ends of the region of interest or beyond the region of interest is needed, such that oligonucleotide primers can be designed; these primers will be identical or similar in sequence to opposite strands of the template to be amplified. The 5' terminal nucleotides of the two primers are in consistence with the ends of the material to be amplified. PCR can be used to amplify specific RNA sequences, specific DNA sequences from total genomic DNA, and cDNA transcribed from total cellular RNA, bacteriophage or plasmid sequences, etc. See generally Mlis et al. (1987) Cold Spring Harbor Symp. Ouant. Biol. 51:263; Erlich, ed., (1989) PCR TECHNOLOGY (Stockton Press, N.Y.). The PCR test used in the present disclosure is considered one, but not the only, example of polymerase reaction method for amplifying a nucleic acid test sample. The method comprises the use of a known nucleic acid as primers and a nucleic acid polymerase to amplify or generate a specific portion of nucleic acid.

"Optional" or "optionally" means that the event or situation that follows may but does not necessarily occur, and the description includes the instances in which the event or situation does or does not occur. For example, "optionally contains 1-3 antibody heavy chain variable regions" means the antibody heavy chain variable region with specific sequence can be, but need not be, present.

"Pharmaceutical composition" refers to a mixture containing one or more compounds according to the present disclosure or a physiologically/pharmaceutically acceptable salt or produg thereof and other chemical components, such as physiologically/pharmaceutically acceptable carriers and excipients. The pharmaceutical composition aims at promoting the administration to an organism, facilitating the absorption of the active ingredient and thereby exerting a biological effect.

### 2. Examples and Test Examples

The following examples are provided to further describe the present disclosure, but are not intended to limit the scope of the disclosure. Experimental methods for which the specific conditions are not indicated in the examples or test examples of the present disclosure are generally carried out according to conventional conditions or according to the conditions recommended by the manufacturer. See Sambrook et al., Molecular Cloning, Laboratory Manual, Cold Spring Harbor Laboratory; Contemporary Molecular Biology Methods, Ausubel et al., Greene Publishing Association, Wiley Interscience, NY. Reagents for which the sources are not specifically indicated are commercially available reagents.

### Example 1: Preparation of reagents such as antigens, related proteins and antibodies

Sequences encoding human interleukin-6 receptor with Fc, His and BP15 tags (used for intracellular site-directed biotinylation), respectively (hIL-6R.Fc, hIL-6R.His, hIL-6R.BP15), sequence encoding cynomolgus IL-6R with Flag.His tag (cIL-6R.FH), sequence encoding human IL-6 with Fc or His tag (IL-6.Fc, IL-6.his), and sequence encoding gp130 with Fc-tag were constructed into expression vector(s) by molecular cloning method, for transient expression in mammalian cell(s).

The amino acid sequences of all antigens and related proteins are as follows:
hIL-6R.His (SEQ ID NO:1) 
LAPRRCPAQEVARGVLTSLPGDSVTLTCPGVEPEDNATVHWVLRKPAAGSHPSRWAGMGRRL
LLRSVQLHDSGNYSCYRAGRPAGTVHLLVDVPPEEPQLSCFRKSPLSNVVCEWGPRSTPSLT
TKAVLLVRKFQNSPAEDFQEPCQYSQESQKFSCQLAVPEGDSSFYIVSMCVASSVGSKFSKT
QTFQGCGILQPDPPANITVTAVARNPRWLSVTWQDPHSWNSSFYRLRFELRYRAERSKTFTT
WMVKDLQHHCVIHDAWSGLRHVVQLRAQEEFGQGEWSEWSPEAMGTPWTESRSPPAENEVST
PMQALTTNKDDDNILFRDSANATSLPVQDSSSVPLPHHHHHH
hIL-6R.BP15 (SEQ ID NO:2) 
LAPRRCPAQEVARGVLTSLPGDSVTLTCPGVEPEDNATVHWVLRKPAAGSHPSRWAGMGRRL
LLRSVQLHDSGNYSCYRAGRPAGTVHLLVDVPPEEPQLSCFRKSPLSNVVCEWGPRSTPSLT
TKAVLLVRKFQNSPAEDFQEPCQYSQESQKFSCQLAVPEGDSSFYIVSMCVASSVGSKFSKT
 
QTFQGCGILQPDPPANITVTAVARNPRWLSVTWQDPHSWNSSFYRLRFELRYRAERSKTFTT
WMVKDLQHHCVIHDAWSGLRHVVQLRAQEEFGQGEWSEWSPEAMGTPWTESRSPPAENEVST
PMQALTTNKDDDNILFRDSANATSLPVQDSSSVPLPGSSDYKDDDDKHHHHHHGLNDIFEAQ
KIEWHE
hIL-6R.Fc (SEQ ID NO:3) 
LAPRRCPAQEVARGVLTSLPGDSVTLTCPGVEPEDNATVHWVLRKPAAGSHPSRWAGMGRRL
LLRSVQLHDSGNYSCYRAGRPAGTVHLLVDVPPEEPQLSCFRKSPLSNVVCEWGPRSTPSLT
TKAVLLVRKFQNSPAEDFQEPCQYSQESQKFSCQLAVPEGDSSFYIVSMCVASSVGSKFSKT
QTFQGCGILQPDPPANITVTAVARNPRWLSVTWQDPHSWNSSFYRLRFELRYRAERSKTFTT
WMVKDLQHHCVIHDAWSGLRHVVQLRAQEEFGQGEWSEWSPEAMGTPWTESRSPPAENEVST
PMQALTTNKDDDNILFRDSANATSLPVQDSSSVPLPEPKSSDKTHTCPPCPAPELLGGPSVF
LFPPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSV
MHEALHNHYTQKSLSLSPGK
cIL-6R.FH (cIL-6R.FlagHis) (SEQ ID NO:4) 
LAPGGCPAQEVARGVLTSLPGDSVTLTCPGGEPEDNATVHWVLRKPAVGSHLSRWAGVGRRL
LLRSVQLHDSGNYSCYRAGRPAGTVHLLVDVPPEEPQLSCFRKSPLSNVVCEWGPRSTPSPT
TKAVLLVRKFQNSPAEDFQEPCQYSQESQKFSCQLAVPEGDSSFYIVSMCVASSVGSKLSKT
QTFQGCGILQPDPPANITVTAVARNPRWLSVTWQDPHSWNSSFYRLRFELRYRAERSKTFTT
WMVKDLQHHCVIHDAWSGLRHVVQLRAQEEFGQGEWSEWSPEAMGTPWTESRSPPAENEVST
PTQAPTTNKDDDNILSRDSANATSLPVQDSSSVPLPGSSDYKDDDDKHHHHHH
IL-6.Fc (SEQ ID NO:5) 
VPPGEDSKDVAAPHRQPLTSSERIDKQIRYILDGISALRKETCNKSNMCESSKEALAENNLN
LPKMAEKDGCFQSGFNEETCLVKIITGLLEFEVYLEYLQNRFESSEEQARAVQMSTKVLIQF
LQKKAKNLDAITTPDPTTNASLLTKLQAQNQWLQDMTTHLILRSFKEFLQSSLRALRQMEPK
SSDKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQ
PREPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK
IL-6.his (SEQ ID NO:6) 
VPPGEDSKDVAAPHRQPLTSSERIDKQIRYILDGISALRKETCNKSNMCESSKEALAENNLN
LPKMAEKDGCFQSGFNEETCLVKIITGLLEFEVYLEYLQNRFESSEEQARAVQMSTKVLIQF
LQKKAKNLDAITTPDPTTNASLLTKLQAQNQWLQDMTTHLILRSFKEFLQSSLRALRQMHHH
HHH
gp130.Fc (SEQ ID NO:7)

ELLDPCGYISPESPVVQLHSNFTAVCVLKEKCMDYFHVNANYIVWKTNHFTIPKEQYTIINR
TASSVTFTDIASLNIQLTCNILTFGQLEQNVYGITIISGLPPEKPKNLSCIVNEGKKMRCEW

DGGRETHLETNFTLKSEWATHKFADCKAKRDTPTSCTVDYSTVYFVNIEVWVEAENALGKVT
SDHINFDPVYKVKPNPPHNLSVINSEELSSILKLTWTNPSIKSVIILKYNIQYRTKDASTWS
QIPPEDTASTRSSFTVQDLKPFTEYVFRIRCMKEDGKGYWSDWSEEASGITYEDRPSKAPSF
WYKIDPSHTQGYRTVQLVWKTLPPFEANGKILDYEVTLTRWKSHLQNYTVNATKLTVNLTND
RYLATLTVRNLVGKSDAAVLTIPACDFQATHPVMDLKAFPKDNMLWVEWTTPRESVKKYILE
WCVLSDKAPCITDWQQEDGTVHRTYLRGNLAESKCYLITVTPVYADGPGSPESIKAYLKQAP
PSKGPTVRTKKVGKNEAVLEWDQLPVDVQNGFIRNYTIFYRTIIGNETAVNVDSSHTEYTLS
SLTSDTLYMVRMAAYTDEGGKDGPEFTFTTPKFAQGEIEEPKSSDKTHTCPPCPAPELLGGP
SVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFS
CSVMHEALHNHYTQKSLSLSPGK

The positive antibodies are: anti-IL-6R humanized mAb tocilizumab available from Roche/Chugai (the sequence is obtained from WO1996011020A1); a fusion protein formed by anti-IL-6R single domain antibody 20A11 and Fc, Ablynx; Sarilumab available from Regeneron (the sequence is obtained from CN101454345B). The amino acid sequences are as follows:
Tocilizumab (Toci) heavy chain (SEQ ID NO:8) 
EVQLQESGPGLVRPSQTLSLTCTVSGYSITSDHAWSWVRQPPGRGLEWIGYISYSGITTYNP
SLKSRVTMLRDTSKNQFSLRLSSVTAADTAVYYCARSLARTTAMDYWGQGSLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEAL
HNHYTQKSLSLSPGK
Tocilizumab (Toci) light chain (SEQ ID NO:9)
20A11-Fc fusion protein (20A11, wherein the VHH sequence is derived from Vobarilizumab) (SEQ ID NO:10) 
EVQLVESGGGLVQPGGSLRLSCAASGSVFKINVMAWYRQAPGKGRELVAGIISGGSTSYADS
VKGRFTISRDNAKNTLYLQMNSLRPEDTAVYYCAFITTESDYDLGRRYWGQGTLVTVSSEPK
SSDKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQ
 
PREPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK
Sarilumab (Sari) heavy chain (SEQ ID NO:11) 
EVQLVESGGGLVQPGRSLRLSCAASRFTFDDYAMHWVRQAPGKGLEWVSGISWNSGRIGYAD
SVKGRFTISRDNAENSLFLQMNGLRAEDTALYYCAKGRDSFDIWGQGTMVTVSSASTKGPSV
FPLAPSSKSTSGGTAALGCLVKDYFPEPVTVSWNSGALTSGVHTFPAVLQSSGLYSLSSVVT
VPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSVMHEALHNH
YTQKSLSLSPGK
Sarilumab (Sari) light chain (SEQ ID NO:12)#

DIQMTQSPSSVSASVGDRVTITCRASQGISSWLAWYQQKPGKAPKLLIYGASSLESGVPSRF
SGSGSGTDFTLTISSLQPEDFASYYCQQANSFPYTFGQGTKLEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC

HEK293E cells (i.e. 293E cells) were transiently transfected with PEI for protein expression. Then the purification method was selected according to the tags or fusion proteins as follows:
1. Nickel Column Purification (Isolation of His-tagged proteins): Supernatants containing the expressed products were high-speed centrifuged to remove impurities. The nickel column was equilibrated with PBS buffer and rinsed with 2-5 column volumes; the supernatant samples were applied to Ni Sepharose Excel Column (GE Healthcare, Cat #17-3712-01) at a certain flow rate. The column was rinsed with PBS buffer until the A280 reading dropped to the baseline, then the column was rinse with PBS + 10 mM imidazole to remove the undesired non-specifically bound proteins and the effluents were collected. Finally, the target proteins were eluted with PBS solution containing 300 mM imidazole and elution peaks were collected.
2. Chromatographic Exclusion Purification: SEC column (superdex75) was pre-equilibrated with PBS. Samples were loaded (the loading volume should be no more than 3% of the column volume) and then eluted with PBS as mobile phase. Each elution peak was collected and the target protein-containing fractions were identified by SDS-PAGE.
3. Flag Affinity Chromatography (Isolation of flag-containing proteins): The flag affinity filler was equilibrated with 0.5 x PBS and washed with 2-5 column volumes. The samples to be purified were incubated with the filler for 2 hours at room temperature. The filler was washed with 5 column volumes of 0.5 x PBS, and the target proteins were eluted with TBS buffer containing 100 µg/ml 3×Flag polypeptide, collected, and identified by SDS-PAGE.
4. Affinity Chromatography of chimeric antibody (Isolation of Fc-containing proteins): Supernatants containing the expressed products were high-speed centrifuged to remove impurities, and the supernatants containing the expressed recombinant antibodies were purified using Protein A column. The column was rinse with PBS until the A280 reading dropped to the baseline. The target proteins were eluted with 100 mM acetic acid, pH 3.0 and neutralized with 1 M Tris-HCI, pH 8.0. The eluted sample was appropriately concentrated and further purified by gel chromatography Superdex 200 (GE) pre-equilibrated with PBS. The peak of the depolymerized product was collected and aliquoted for use.

### Example 2: Lama immunization, phage display library construction and screening

IL-6R.Fc or IL-6R.His, used as an immunogen, was mixed with Freund's complete adjuvant or Freund's incomplete adjuvant or PBS, and then was injected into alpacas 6 times. Peripheral blood samples were taken for titer determination before and after of 4^{th}, 5^{th} and 6^{th} immunizations. Splenocytes were extracted after the last immunization.

The isolated lymphocytes were subjected to RNA extraction followed by reverse transcription into cDNAs. Two-step PCR was used for library construction: in first step, three forward primers designed on FR1 region and three reverse primers designed on CH2 region were mixed at equal ratio for PCR reaction. Small fragments were recovered from agarose gel. In the second step, both the forward and reverse primers were incorporated with Sfil restriction site and protective base. Except for the Sfil restriction site and the protective base, the sequences of forward primers were identical to the forward primers used in the first round PCR. The reverse primers were designed on FR4 region. The second step PCR products were recovered and cloned into phagemid vector, then electro-transformed into SS320 competent cells. Finally, a phage display library with capacity of 1.74E9 was obtained.

The constructed library was packaged with a helper phage to form a phage. The phage display library was panned and screened for IL-6R binding. Desired clones were identified by ELISA. The panning was performed by both liquid phase and solid phase method: in the liquid phase method, biotinylated IL-6R.BP15 antigen bind to phage particles in the liquid phase and then were captured by streptavidin magnetic beads; in the solid phase method, ELISA plate was coated with IL-6R.Fc which was bound to phage particles. After two rounds of panning, monoclonal packed phages were picked up. The binding activity and the IL-6- and gp130-blocking activity, were determined by ELISA as follows:
IL-6R-binding ELISA: The plate was coated with 2 ng/µl streptavidin, 100 µl/well, overnight at 4°C, and blocked with 2% MPBSCaMg at 37°C for 1 hour. Then the plate was washed and incubated with 1 ng/µl of IL-6R.BP15 at 37°C for 1 hour. The plate was then washed followed by addition of 50 µl/well of 2% MPBSCaMg and 50 µl/well of phage supernatant. After Incubation at 37°C for 1 hour, the plate was washed followed by addition of 100 µl of anti-M13 HRP with1:5000 dilution. After 1-hour incubation at 37°C and wash, 100 µl/well of TMB was added for plate development. The reaction was stopped by addition of 100 µl of 1 M H₂SO₄. And signal were determined by at OD₄₅₀.

IL-6-blocking ELISA: The plate was coated with 100ul/well of IL-6.Fc at 2 ng/µl. After overnight incubation at 4°C, the plate was blocked with 2% MPBSCaMg (2% milk, 0.90mM CaCl₂, 0.49mM MgCl₂, 1×PBS) at 37°C for 1 hour. After washing, the plate was incubated with 100 µl of IL-6R.his (1 ng/µl) at 37°C for 1 hour. Then, the plate was washed and incubated with 50 µl/well of phage supernatant + 50 µl/well of 2% MPBSCaMg blocking solution at 37°C for 1 hour. The plate was then washed followed by incubation with 100 µl of anti-M13 HRP (GE healthcare, Cat# 27-9421-01), diluted at 1:5000, at 37°C for 1 hour. After washing and TMB incubation, the reaction was stopped by addition of 100 µl of 1 M H₂SO₄. And signal was read at OD₄₅₀.

Gp130-blocking ELISA: The plate was coated with 2 ng/µl gp130, 100 µl/well, overnight at 4°C, and blocked with 2% MPBSCaMg at 37°C for 1 hour. Then, the plate was washed and incubated with 100µl 1ng/µl IL-6R.his + 1 ng/µl of IL-6R.his at 37°C for 1 hour. After washing, 50 µl/well of phage supernatant + 50 µl/well of 2% MPBSCaMg blocking solution were added followed by 1hour incubation at 37°C for 1 hour. Then, the plate was washed and 100 µl of anti-M13 HRP, diluted at 1:5000, was added in each well followed by incubation at 37°C for 1 hour. After washing, the plate was developed with 100µl/well of TMB. The reaction was stopped by addition of 100 µl of 1 M H₂SO₄, and the signal was measured at OD₄₅₀.

Clones that meet preselected criteria were selected for sequencing, such as: positive IL-6R-binding result, the reading of OD₄₅₀ (IL-6R-binding ELISA)/OD₄₅₀ (IL-6-blocking ELISA) (IL-6R/IL-6) was equal or higher than 4; the reading of OD₄₅₀ (IL-6-blocking ELISA)/OD₄₅₀ (IL-6-blocking ELISA) (IL-6/gp130) was equal or greater than 3. The sequences were analyzed and a panel of clones with unique sequences was identified

### Example 3: Construction and characterization of Fc fusion proteins

In order to further characterize the selected clones at molecular and cellular level, the identified VHHs were fused to N-terminus of Fc, and cloned into mammalian expression vectors followed by transiently expressed in 293E cells. After protein A purification, VHH-hFc fusion proteins were subjected to the following tests.

IL-6R-blocking assay (Test Example 1), gp130-binding blocking assay (Test Example 2), U266B1 cell binding activity (Test Example 3), and IL-6-stimulated TF-1 proliferation blocking assay (Test Example 4).

An IL-6R single domain antibody, 1764, which has the best properties in all aspects was selected. Its nucleotide sequence is shown below: (SEQ ID NO:13) 
gaggtgcagctggtggagtctgggggaggttggtgcaggctgggggtctctgagactctc
ctgtgaagcctctggaaacatcttcaagatcaatgtcatgggctggtaccgccaggctccag
 
ggaagcagcgcgagtgggtcgcagctattattagtggcggtagcacaaactatgcagactcc
gtgaagggccgattcaccatctccagagacaacgccaagaacacggtgtatctgcaaatgaa
cagcctgaaacctgaggacacggccgtctattactgtaatgctattctcacctataacgact
atgacctagggtctgactactggggccaggggacccaggtcaccgtctcctca
 The encoded amino acid sequence is shown below: (SEQ ID NO:14) 
EVQLVESGGGLVQAGGSLRLSCEASGNIFKINVMGWYRQAPGKQREWVAAIISGGSTNYADS
VKGRFTISRDNAKNTVYLQMNSLKPEDTAVYYCNAILTYNDYDLGSDYWGQGTQVTVSS
 wherein CDR 1-3 of the single domain antibody 1764 are underlined, and the sequences are:

| | | |
|---|---|---|
| CDR1: | INVMG | (SEQ ID NO:15) |
| CDR2: | AIISGGSTNYADSVKG | (SEQ ID NO:16) |
| CDR3: | ILTYNDYDLGSDY | (SEQ ID NO:17) |

The above single domain antibody VHH was fused to the following Fc fragment to form a fusion protein. Nucleotide sequence encoding the human Fc fragment is shown as follow: (SEQ ID NO:18)

gagcccaaatctagtgacaaaactcacacgtgcccaccgtgcccagcacctgaactcctggg
gggaccgtcagtcttcctcttccccccaaaacccaaggacaccctcatgatctcccggaccc
ctgaggtcacatgcgtggtggtggacgtgagccacgaagaccctgaggtcaagttcaactgg
tacgtggacggcgtggaggtgcataatgccaagacaaagccgcgggaagagcagtacaacag
cacgtaccgtgtggtcagcgtcctcaccgtcctgcaccaggactggctgaatggcaaggagt
acaagtgcaaggtctccaacaaagccctcccagcccccatcgagaaaaccatctccaaagcc
aaagggcagccccgagaaccacaggtgtacaccctgcccccatcccgggatgagctgaccaa
gaaccaggtcagcctgacctgcctggtcaaaggcttctatcccagcgacatcgccgtggagt
gggagagcaatgggcagccggagaacaactacaagaccacgcctcccgtgctggactccgac
ggctccttcttcctctacagcaagctcaccgtggacaagagcaggtggcagcaggggaacgt
cttctcatgctccgtgatgcatgaggctctgcacaaccactacacgcagaagagcctctccc
tgtctccgggtaaatga

The human Fc fragment: (SEQ ID NO:19)

EPKSSDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK

### Example 4: Humanization of 1764

In order to avoid the anti-drug response of the therapeutic antibody, the lama-derived molecule 1764 was humanized in the present disclosure to reduce its immunogenicity. Humanization was based on the sequences of 1764 and performed in a manner of gradual mutation toward human sequence, and the lama-derived amino acid residues located on FR region of 1764 were gradually replaced with human-derived residues. The designed sequences are as follows.

**Table 1: The designed sequences**

| **Variant No.** | **Mutation sites in 1764 variants** |
|---|---|
| 1 | A14P, K86R |
| 2 | E23A, Q44G |
| 3 | A14P, K86R, Q116L |
| 4 | E23A, Q44G, Q116L |
| 5 | E23A, N96A, A97F, Q116L |
| 6 | A14P, E23A, Q44G, V78L, K86R, Q116L |
| 7 | A14P, E23A, V78L, K86R, N96A, A97F, Q116L |
| 8 | E23A, Q44G, V78L, N96A, A97F, Q116L |
| 9 | A14P, E23A, Q44G, V78L, K86R, N96A, A 97F, Q116L |

| | |
|---|---|
| Note: For example, A14P indicates that the amino acid residue A at position 14 of 1764 was substituted by P. The numbering of the amino acid residues in this table is according to the natural position numbering as shown in SEQ ID NO: 14. | |

1764-mu1 (SEQ ID NO: 20) 
EVQLVESGGGLVQPGGSLRLSCEASGNIFKINVMGWYRQAPGKQREWVAAIISGGSTNYADS
VKGRFTISRDNAKNTVYLQMNSLRPEDTAVYYCNAILTYNDYDLGSDYWGQGTQVTVSS
1764-mu2 (SEQ ID NO: 21) 
EVQLVESGGGLVQAGGSLRLSCAASGNIFKINVMGWYRQAPGKGREWVAAIISGGSTNYADS
VKGRFTISRDNAKNTVYLQMNSLKPEDTAVYYCNAILTYNDYDLGSDYWGQGTQVTVSS
1764-mu3 (SEQ ID NO: 22) 
EVQLVESGGGLVQPGGSLRLSCEASGNIFKINVMGWYRQAPGKQREWVAAIISGGSTNYADS
VKGRFTISRDNAKNTVYLQMNSLRPEDTAVYYCNAILTYNDYDLGSDYWGQGTLVTVSS
1764-mu4 (SEQ ID NO: 23) 
EVQLVESGGGLVQAGGSLRLSCAASGNIFKINVMGWYRQAPGKGREWVAAIISGGSTNYADS
VKGRFTISRDNAKNTVYLQMNSLKPEDTAVYYCNAILTYNDYDLGSDYWGQGTLVTVSS
1764-mu5 (SEQ ID NO: 24) 
EVQLVESGGGLVQAGGSLRLSCAASGNIFKINVMGWYRQAPGKQREWVAAIISGGSTNYADS
VKGRFTISRDNAKNTVYLQMNSLKPEDTAVYYCAFILTYNDYDLGSDYWGQGTLVTVSS
1764-mu6 (SEQ ID NO: 25) 
EVQLVESGGGLVQPGGSLRLSCAASGNIFKINVMGWYRQAPGKGREWVAAIISGGSTNYADS
VKGRFTISRDNAKNTLYLQMNSLRPEDTAVYYCNAILTYNDYDLGSDYWGQGTLVTVSS
1764-mu7 (SEQ ID NO: 26) 
EVQLVESGGGLVQPGGSLRLSCAASGNIFKINVMGWYRQAPGKQREWVAAIISGGSTNYADS
VKGRFTISRDNAKNTLYLQMNSLRPEDTAVYYCAFILTYNDYDLGSDYWGQGTLVTVSS
1764-mu8 (SEQ ID NO: 27) 
EVQLVESGGGLVQAGGSLRLSCAASGNIFKINVMGWYRQAPGKGREWVAAIISGGSTNYADS
VKGRFTISRDNAKNTLYLQMNSLKPEDTAVYYCAFILTYNDYDLGSDYWGQGTLVTVSS
1764-mu9 (SEQ ID NO: 28)

EVQLVESGGGLVQPGGSLRLSCAASGNIFKINVMGWYRQAPGKGREWVAAIISGGSTNYADS
VKGRFTISRDNAKNTLYLQMNSLRPEDTAVYYCAFILTYNDYDLGSDYWGQGTLVTVSSEPK
SS

The designed nine clones were fused to Fc (amino acid sequence was shown in SEQ ID NO:19), constructed into mammalian transient expression vectors, and transiently expressed in 293E cells. After affinity purification, VHH-hFc fusion protein was obtained. The purified IL-6R antibody fusion proteins (containing Fc fragment, hereinafter also referred to as IL-6R antibodies) were subjected to the following tests.

### Test Example 1: IL-6R Blocking Assay with IL-6R Antibodies

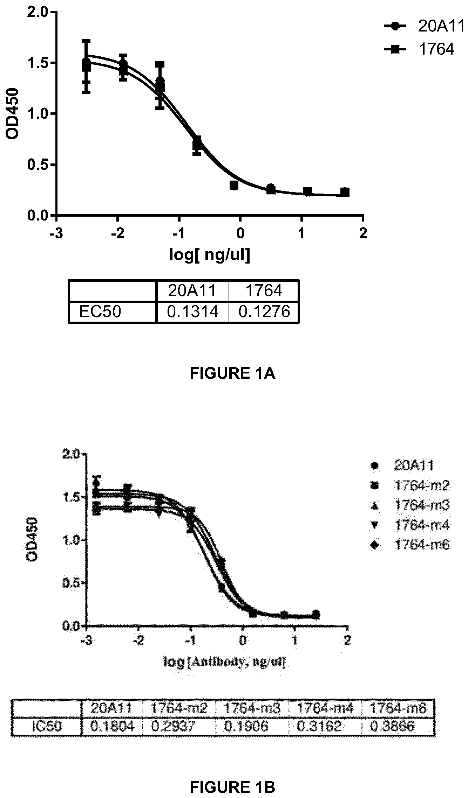

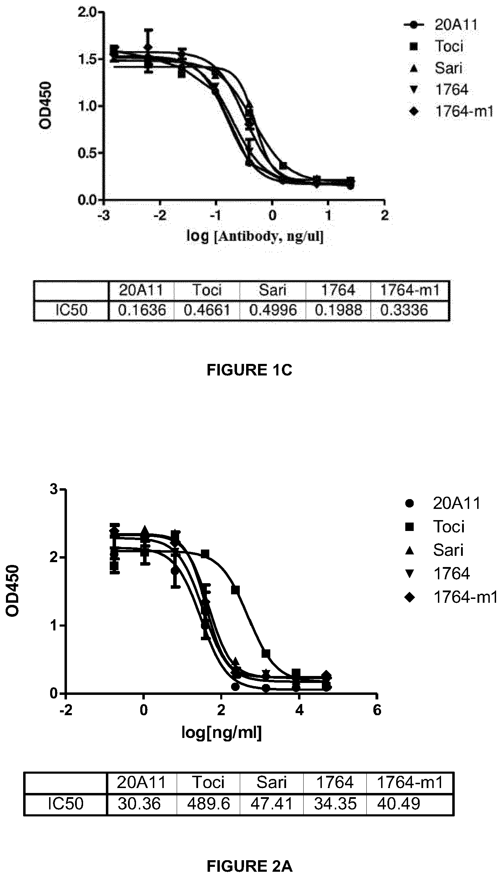

The formation of IL-6R/IL-6 complex can be blocked by the binding of IL-6R antibody to IL-6R, indicating the downstream cell signaling could be blocked by preventing IL-6/IL-6R complex formation on the cell membrane. All of the expressed the purified antibodies were tested for their abilities to block the binding between IL-6 and IL-6R by ELISA. The procedures were as follows:
The plates were coated with 100 µl of 2 ng/µl IL-6.Fc at 4°C overnight, and blocked with 2% skim milk at 37°C for 1 hour. The plates were then washed, incubated with 100 µl of serial diluted testing samples with and 1 ng/µl IL-6R.BP15. After 1-hour incubation at 37°C, the plates were washed with HRP-streptavidin (Jackson, 016-030-084, diluted at 1:4000) was added to the plate followed by incubation. After washing and adding 100 µl of TMB the plates were developed at room temperature for 5 min. 100 µl of 1M H₂SO₄ was added to terminate the reaction. The OD₄₅₀ values were read. The results are shown in Figures 1A-C).

### Test Example 2: gp130-Binding Blocking Assay with IL-6R Antibodies

The complex of IL-6 and IL-6R can bind to GP130. The assay will evaluate if the selected antibodies were able to block the formation of IL-6/IL-6R/gp130 complex by binding to IL-6R. The plates were coated with 1 µg/ml gp130 (diluted in PBS) at 4°C overnight. The coating solution was discarded, and plates were blocked with 5% skim milk in PBS at 37°C for 2-3h. After blocking, the plates were washed 3 times with washing buffer PBST (0.1% Tween-20 in PBS).

IL-6.his and IL-6R.BP15 were diluted with 1% BSA in PBS to a concentration of 0.06 µg/ml. Starting at 50 µg/ml, IL-6R antibodies were 1:3 serially diluted in dilution buffer and Both testing antibody samples and IL-6/IL-6R complex were added to the plate followed by 1h incubation at 37°C. Then, the plates were washed for 3 times with washing buffer PBST (0.1% Tween-20 in PBS).

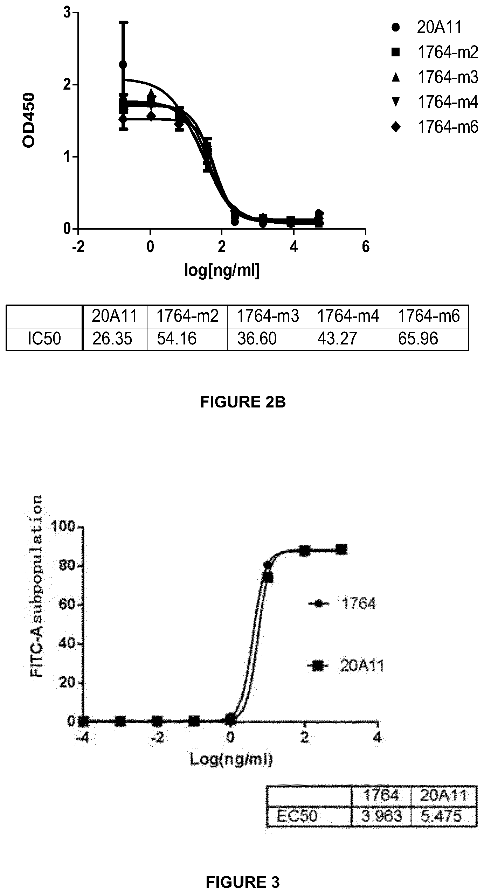

1:2000 diluted Secondary antibody HRP streptavidin (Jackson, 016-030-084) was added to the plate followed by 1h incubation at 37°C. After incubation and washing, TMB was used for development. OD₄₅₀ values were read. The results are shown in Figures 2A-B.

### Test Example 3: Binding activities of IL-6R antibodies to U266B1 cells

To test binding abilities of IL-6R antibodies to cell surface IL-6R, binding assay was performed on IL-6R-expressing U266B1 cells with IL-6R antibodies. U266B1 cells (ATCC® TIB-196™) were collected, centrifuged at 400g and 4°C for 5min. Pre-cooled DPBS containing final concentration of 10% FBS was used to resuspend cell pellet followed by centrifuged at 400g, 4°C for 5min. After two washings cells were seeded onto 96-well plates, 10E⁵/well. Each well was loaded with 50 µl sample, and incubated at 4 °C for 45-60 minutes followed by addition of 250 µl pre-cooled DPBS containing final concentration of 10% FBS. After two washings 50 µl secondary antibody Alexa Fluor@488 goat anti-human IgG (H+L) (Life Technologies, A11013, diluted at 1:200) were added and incubated at 4°C in the darkness for 45 minutes. Then, the plates were washed with 250 µl/well of pre-cooled DPBS containing final concentration of 10% FBS for two times. To each well, 100 µl/well of pre-cooled DPBS were added for cell resuspension. Detection was performed on machine (BD, FACSverse). Results were analyzed with FlowJo software and shown in Figure 3.

### Test Example 4: Inhibition of IL-6-stimulated TF-1 proliferation by IL-6R antibodies

Through binding to membrane-type IL-6R, IL-6 can initiate downstream signaling and induce proliferation of TF-1 cells. IL-6R antibodies can block the binding of IL-6 to IL-6R, thereby inhibit the proliferation of TF-1 cells.

TF-1 cell culture: TF-1 leukemia cells (ATCC, CRL-2003) were cultured in RPMI1640 containing 10% FBS (2ng/ml rhGM-CSF) (GE, Catalog No. SH30809.01), and placed in incubator at 37°C, 5% CO₂, at the cell density not above 10⁶ cells/ml.

The procedures of assay for inhibiting IL-6-stimulated TF-1 proliferation: Cells in logarithmic growth phase were washed three times with PBS, centrifuged at 800 rpm for 3 min, and the cell density was adjusted with RPMI1640 (FBS 2%, recombinant human IL-6, 5ng/ml) to 20,000 cells/well/180 µl. 20 µl of serial dilutions of the test antibodies were added to 96-well plates and incubated for 3 days (the starting antibody concentration of the serial dilution was 490 nM, and 10-fold serially diluted to 4.9×10⁻⁷ nM). 100 µl of cell suspension was mixed with 100 µl of Cell Titer (Promega, Catalog No. G7573) for detection. The results are shown in Table 2.

**Table 2: The blocking effect of IL-6R antibodies on IL-6-stimulated TF-1 proliferation**

| | 20A11 | 1764 | 1764-mu1 | 1764-mu2 | 1764-mu3 | 1764-mu4 | 1764-mu6 |
|---|---|---|---|---|---|---|---|
| EC50 (nM) | 0.3545 | 0.4100 | 0.4469 | 0.3442 | 0.3365 | 0.3704 | 0.6032 |

### Test Example 5: IL-6R antibodies inhibit IL-6-induced hepcidin expression

High expression of hepcidin is major cause of inflammatory anemia. IL-6 will up-regulate hepcidin expression by addition of IL-6R to activate the downstream signaling pathways. Therefore, IL-6R antibodies may inhibit IL-6-induced hepcidin expression by blocking the binding between IL-6 and IL-6R. The experimental design is as follows:
Group 1: blank control
Group 2: positive control, treated with IL-6 only
Group 3: treated with 1764-mu3 and IL-6
Group 4: treated with Tocilizumab and IL-6

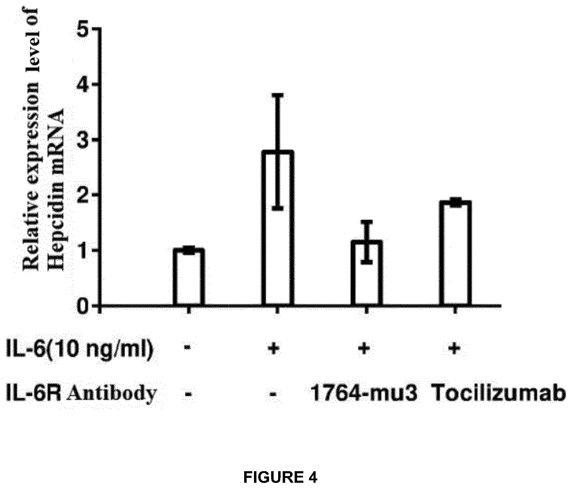

Hep3B cells (Chinese Academy of Sciences, TCHu106) were cultured in EME medium (Gibco, 11095098) containing 10% FBS, and incubated in an incubator at 37°C, 5% CO₂. Cell passage was performed every 2 to 3 days. Hep3B cells in logarithmic growth phase were plated in 6-well plates at 200,000 cells per well and cultured in an incubator at 37°C, 5% CO₂. On the next day, when cell confluency reached 80% to 90%, IL-6R antibody 1764-mu3 and Tocilizumab were added to each well in Group 3 and Group 4, respectively, at a final concentration of 5 nM. After incubation in an incubator at 37°C, 5% CO₂ for 30 min, each well was added with IL-6 protein at a final concentration of 10 ng/ml, except for the blank control. The cells were further cultivated for 24 hours, and then the cells were collected, total RNA was extracted, and reverse transcribed into cDNA. Fluorescent quantitative PCR was performed by using cDNA as a template to detect the mRNA level of hepcidin, with the expression level of microglobulin mRNA as an internal reference. The results are shown in Table 3 and Figure 4.

**Table 3: The inhibition of IL-6R antibodies on IL-6-induced hepcidin expression level**

| Sample Name | Group 1 | Group 2 | Group 3 | Group 4 |
|---|---|---|---|---|
| Relative expression level of hepcidin mRNA (fold) | 1 | 2.783 | 1.154 | 1.866 |

### Test Example 6: BIAcore Affinity (KD) Assay of the Antibodies

Human anti-capture antibodies were covalently coupled onto Biosensor chip CM5 (Cat.# BR-1000-12, GE) in a BIAcore instrument (BIAcore T200, GE) with Human Anti-Capture Kit (Cat. #BR-1008-39, GE) according to manufacturer's protocol, thereby certain amounts of antibodies to be tested were captured. Antigens (hIL-6R.his and cIL-6R.FH) at a series of concentration gradients were then flowed through the surface of the chip. The reaction signals were detected in real time using BIAcore instrument (BIAcore T200, GE) to obtain binding and dissociation curves.

After each cycle of dissociation, the biochips were washed and regenerated with the regeneration solution provided in the Human Anti-Capture Kit. The amino coupling kit used in the experiment was purchased from GE (Cat. #BR-1000-50, GE) and the buffer was HBS-EP+10×buffer solution (Cat.# BR-1006-69, GE), diluted to 1 × (pH 7.4) with D.I. water. The experimental data were fitted with BIA evaluation version 4.1 and GE software (1:1) Langmuir model to obtain the affinity values. The results are shown in Table 4.

**Table 4: Afinity of 1764 and each humanized clone for hIL-6R detected in BIAcore sssay**

| sample | hIL-6R | | |
|---|---|---|---|
| | **ka (1/Ms)** | **kd (1/s)** | **KD (M)** |
| 1764 | 1.28E+06 | 1.64E-04 | 1.29E-10 |
| 1764-mu1 | 1.23E+06 | 1.28E-04 | 1.05E-10 |
| 1764-mu2 | 1.23E+06 | 1.54E-04 | 1.26E-10 |
| 1764-mu3 | 1.21E+06 | 1.54E-04 | 1.27E-10 |
| 1764-mu4 | 1.20E+06 | 1.50E-04 | 1.25E-10 |
| 1764-mu5 | 1.10E+06 | 1.11E-04 | 1.01E-10 |
| 1764-mu6 | 1.23E+06 | 2.45E-04 | 2.00E-10 |
| 1764-mu7 | 8.97E+05 | 1.17E-04 | 1.31E-10 |
| 1764-mu8 | 1.01E+06 | 1.44E-04 | 1.43E-10 |
| 1764-mu9 | 8.53E+05 | 1.21E-04 | 1.42E-10 |

### Test Example 7: Pharmacokinetic experiment in rats

Pharmacokinetic experiment of antibody fusion proteins were performed in rats. The dosing schedule to rats were as follows:

**Table 5. Dosing schedule**

| Animal Group Number | B | C |
|---|---|---|
| Compound Name | 1764-mu3 | 1764-mu4 |
| Dosing route | i.v. | i.v. |
| Formulation | PBS, pH7.4, saline | |
| Administration Dosage (mg/kg) | 5 | 5 |
| Administration Volume (ml/kg) | 5 | 5 |
| Drug Concentration (mg/ml) | 1 | 1 |

12 SD rats (Sipper-Beikai Experimental Animal Co., Ltd.) (equal number of male and female) were divided into 2 groups, 6 rats in each group. The drugs were intravenously injected. In treatment group, 0.2 ml of whole blood samples were collected without addition of anticoagulant before administration as well as 15 min, 8 h, 1 day, 2 days, 4 days, 7 days, 10 days, 14 days, 21 days and 28 days of post administration. Blood samples were placed at 4°C for 30 min, centrifuged at 1000 g for 15 min, and the supernatants (serum) were placed in EP tubes and stored at -80°C. The concentration of the target was determined by ELISA, and the pharmacokinetic parameters of the test drugs were calculated using Winnolin software. The test results are shown in Table 6.

**Table 6. Pharmacokinetic parameters**

| | 1764-mu3 | 1764-mu4 |
|---|---|---|
| dosage | 5mg/kg | 5mg/kg |
| Tₘₐₓ (h) | 0.25 | 0.25 |
| t_{1/2} (h) | 185±17 | 148±9 |

1764-mu3 and 1764-mu4 are single-chain antibodies with half-lives of 7.7 days and 6.2 days, respectively.

### Test Example 8: Test for Accelerated Stability

In order to observe the stability of the antibodies, an accelerated test was performed on some antibodies. 1 mg/ml antibody fusion protein was placed at 40°C for 3 days, and then was observed for the presence or absence of precipitation, and were subjected to SEC detection. The test results are shown in Table 7.

**Table 7. Accelerated experimental results**

| **clone** | **SEC purity( %)** | **40°C for 3days** |
|---|---|---|
| 1764 | 96.97 | clear |
| 1764-mu1 | 98.09 | clear |
| 1764-mu2 | 95.84 | clear |
| 1764-mu3 | 98.21 | clear |
| 1764-mu4 | 94.98 | clear |

For clear understanding, the above invention has been described in detail with the aid of the accompanying drawings and examples. However, the description and examples should not be construed as limiting the scope of the disclosure. The numerical units in all figures are the same as the units indicated on the abscissa. All patents and scientific literatures cited herein are expressly incorporated by reference in their entirety.

## Claims

1. A monoclonal antibody or antigen-binding fragment thereof comprising CDR1, CDR2 and CDR3, wherein:
the sequence of CDR1 is selected from the group consisting of the sequence set forth in SEQ ID NO:15 and sequence variants having 3, 2 or 1 amino acid difference from the sequence set forth in SEQ ID NO:15;
the sequence of CDR2 is selected from the group consisting of the sequence set forth in SEQ ID NO:16 and sequence variants having 3, 2 or 1 amino acid difference from the sequence set forth in SEQ ID NO:16;
the sequence of CDR3 is selected from the group consisting of the sequence set forth in SEQ ID NO:17 and sequence variants having 3, 2 or 1 amino acid difference from the sequence shown in SEQ ID NO:17;
the monoclonal antibody or antigen-binding fragment thereof binds to human IL-6R.

2. The monoclonal antibody or antigen-binding fragment thereof according to claim 1, wherein the CDR1 sequence is set forth in SEQ ID NO:15, the CDR2 sequence is set forth in SEQ ID NO:16, and the CDR3 sequence is set forth in SEQ ID NO:17.

3. The monoclonal antibody or antigen-binding fragment thereof according to claim 1 or 2, wherein the monoclonal antibody is a recombinant antibody, and preferably selected from the group consisting of alpaca-derived antibodies, chimeric antibodies, and humanized antibodies; and preferably the monoclonal antibody comprises VHH set forth in SEQ ID NO:14.

4. The monoclonal antibody or antigen-binding fragment thereof according to claim 3, wherein the humanized antibody comprises variant of VHH set forth in SEQ ID NO:14.

5. The monoclonal antibody or antigen-binding fragment thereof according to claim 4, wherein the variant of the VHH sequence has 1 to 15 humanized amino acid mutation(s) acid in FR region of the VHH set forth in SEQ ID NO:14; preferably, the mutation of amino acid is selected from the group consisting of A14P, E23A, Q44G, V78L, K86R, N96A, A97F, Q116L or combinations thereof on VHH as set forth in SEQ ID NO: 14; most preferably, the amino acid sequence of the variant is selected from group consisting of SEQ ID NOs:20 to 28.

6. The monoclonal antibody or antigen-binding fragment thereof according to any one of claims 1 to 5, wherein the antibody further comprises a human antibody Fc region, preferably the antibody comprises a human antibody Fc region set forth in SEQ ID NO:19.

7. The monoclonal antibody or antigen-binding fragment thereof according to any one of claims 1 to 6, wherein the antigen-binding fragment is a single domain antibody or a peptide comprising the three CDRs.

8. An isolated monoclonal antibody or antigen-binding fragment thereof, which competes with the monoclonal antibody or antigen-binding fragment thereof according to any one of claims 1 to 7 for binding to IL-6R and blocks the binding of IL-6 to IL-6R.

9. A pharmaceutical composition comprising a therapeutically effective amount of the monoclonal antibody or antigen-binding fragment thereof according to any one of claims 1 to 8, and one or more pharmaceutically acceptable carriers, diluents or excipients.

10. A nucleic acid molecule encoding the monoclonal antibody or antigen-binding fragment thereof according to any one of claims 1 to 8.

11. The nucleic acid molecule of claim 10 comprising a polynucleotide having at least 80% homology to the nucleic acid molecule as set forth in SEQ ID NO:13.

12. A recombinant vector comprising the nucleic acid molecule according to claim 10 or 11.

13. A host cell, transformed with the recombinant vector according to claim 12, the host cell is a prokaryotic cell or a eukaryotic cell, preferably, the host cell is a eukaryotic cell; more preferably, the host cell is a mammalian cell or a yeast cell.

14. A method for producing the monoclonal antibody or antigen-binding fragment thereof according to any one of claims 1 to 8, the method comprises the steps of:
cultivating the host cell according to claim 13;
forming and accumulating the monoclonal antibody or antigen-binding fragment thereof according to any one of claims 1 to 8 in culture; and
recovering the monoclonal antibody or antigen-binding fragment thereof from the culture.

15. A method for detecting or determining human IL-6R, comprising the step of contacting the monoclonal antibody or antigen-binding fragment thereof according to any one of claims 1 to 8 with a sample.

16. An agent for detecting or determining human IL-6R, comprising the monoclonal antibody or antigen-binding fragment thereof according to any one of claims 1 to 8.

17. Use of the monoclonal antibody or antigen-binding fragment thereof according to any one of claims 1 to 8, or the pharmaceutical composition according to claim 9, or the nucleic acid molecule according to claim 10 or 11, in the preparation of a medicament for the treatment or prevention of a disease associated with human IL-6R.

18. Use of the monoclonal antibody or antigen-binding fragment thereof according to any one of claims 1 to 8, or the pharmaceutical composition according to claim 9, or the nucleic acid molecule of claim 10 or 11, or the combination thereof, in the preparation of a medicament for the treatment or prevention of one or more diseases selected from the group consisting of sepsis, multiple myeloma, renal cell carcinoma, plasma cell leukemia, lymphoma, B-lymphoid hyperplasia, prostate cancer, osteoporosis, cachexia, psoriasis, glomerular mesangial proliferative glomerulonephritis, Kaposi's sarcoma, AIDS-related lymphoma, rheumatoid arthritis, systemic onset of juvenile idiopathic arthritis, hypergammaglobulinemia, regional enteritis, ulcerative colitis, systemic lupus erythematosus, multiple sclerosis, Castleman's disease, IgMy-globulin disease, cardiac myxoma, asthma, autoimmune insulin-dependent diabetes and inflammatory anemia.


Sequence listing

<110>  JIANGSU HENGRUI MEDICINE CO.,LTD; SHANGHAI HENGRUI PHARMACEUTICAL
CO.,LTD

<120>  IL-6R ANTIBODY AND ANTIGEN-BINDING FRAGMENT THEREOF AND MEDICAL USE

<130>  780070CPCT

<160>  28

<170>  PatentIn version 3.3

<210>  1
<211>  352
<212>  PRT
<213>  artificial sequence

<220>
<223>  hIL-6R.His

<400>  1

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Asp Ser Ser Ser Val Pro Leu Pro His His His His His His
            340                 345                 350

<210>  2
<211>  378
<212>  PRT
<213>  artificial sequence

<220>
<223>  hIL-6R.BP15

<400>  2

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser

210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Asp Ser Ser Ser Val Pro Leu Pro Gly Ser Ser Asp Tyr Lys
            340                 345                 350

Asp Asp Asp Asp Lys His His His His His His Gly Leu Asn Asp Ile
        355                 360                 365

Phe Glu Ala Gln Lys Ile Glu Trp His Glu
    370                 375

<210>  3
<211>  578
<212>  PRT
<213>  artificial sequence

<220>
<223>  hIL-6R.Fc

<400>  3

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu

20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Asp Ser Ser Ser Val Pro Leu Pro Glu Pro Lys Ser Ser Asp
            340                 345                 350

Lys Thr His Thr Cys Pro Pro Cys Pro Ala Pro Glu Leu Leu Gly Gly
        355                 360                 365

Pro Ser Val Phe Leu Phe Pro Pro Lys Pro Lys Asp Thr Leu Met Ile
    370                 375                 380

Ser Arg Thr Pro Glu Val Thr Cys Val Val Val Asp Val Ser His Glu
385                 390                 395                 400

Asp Pro Glu Val Lys Phe Asn Trp Tyr Val Asp Gly Val Glu Val His
                405                 410                 415

Asn Ala Lys Thr Lys Pro Arg Glu Glu Gln Tyr Asn Ser Thr Tyr Arg
            420                 425                 430

Val Val Ser Val Leu Thr Val Leu His Gln Asp Trp Leu Asn Gly Lys
        435                 440                 445

Glu Tyr Lys Cys Lys Val Ser Asn Lys Ala Leu Pro Ala Pro Ile Glu
    450                 455                 460

Lys Thr Ile Ser Lys Ala Lys Gly Gln Pro Arg Glu Pro Gln Val Tyr
465                 470                 475                 480

Thr Leu Pro Pro Ser Arg Asp Glu Leu Thr Lys Asn Gln Val Ser Leu

485                 490                 495

Thr Cys Leu Val Lys Gly Phe Tyr Pro Ser Asp Ile Ala Val Glu Trp
            500                 505                 510

Glu Ser Asn Gly Gln Pro Glu Asn Asn Tyr Lys Thr Thr Pro Pro Val
        515                 520                 525

Leu Asp Ser Asp Gly Ser Phe Phe Leu Tyr Ser Lys Leu Thr Val Asp
    530                 535                 540

Lys Ser Arg Trp Gln Gln Gly Asn Val Phe Ser Cys Ser Val Met His
545                 550                 555                 560

Glu Ala Leu His Asn His Tyr Thr Gln Lys Ser Leu Ser Leu Ser Pro
                565                 570                 575

Gly Lys

<210>  4
<211>  363
<212>  PRT
<213>  artificial sequence

<220>
<223>  cIL-6R.FH

<400>  4

Leu Ala Pro Gly Gly Cys Pro Ala Gln Glu Val Ala Arg Gly Val Leu
1               5                   10                  15

Thr Ser Leu Pro Gly Asp Ser Val Thr Leu Thr Cys Pro Gly Gly Glu
            20                  25                  30

Pro Glu Asp Asn Ala Thr Val His Trp Val Leu Arg Lys Pro Ala Val
        35                  40                  45

Gly Ser His Leu Ser Arg Trp Ala Gly Val Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu

85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Pro Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Leu Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Thr Gln Ala Pro Thr Thr Asn Lys Asp
305                 310                 315                 320

Asp Asp Asn Ile Leu Ser Arg Asp Ser Ala Asn Ala Thr Ser Leu Pro
                325                 330                 335

Val Gln Asp Ser Ser Ser Val Pro Leu Pro Gly Ser Ser Asp Tyr Lys
            340                 345                 350

Asp Asp Asp Asp Lys His His His His His His
        355                 360

<210>  5
<211>  415
<212>  PRT
<213>  artificial sequence

<220>
<223>  IL-6.Fc

<400>  5

Val Pro Pro Gly Glu Asp Ser Lys Asp Val Ala Ala Pro His Arg Gln
1               5                   10                  15

Pro Leu Thr Ser Ser Glu Arg Ile Asp Lys Gln Ile Arg Tyr Ile Leu
            20                  25                  30

Asp Gly Ile Ser Ala Leu Arg Lys Glu Thr Cys Asn Lys Ser Asn Met
        35                  40                  45

Cys Glu Ser Ser Lys Glu Ala Leu Ala Glu Asn Asn Leu Asn Leu Pro
    50                  55                  60

Lys Met Ala Glu Lys Asp Gly Cys Phe Gln Ser Gly Phe Asn Glu Glu
65                  70                  75                  80

Thr Cys Leu Val Lys Ile Ile Thr Gly Leu Leu Glu Phe Glu Val Tyr
                85                  90                  95

Leu Glu Tyr Leu Gln Asn Arg Phe Glu Ser Ser Glu Glu Gln Ala Arg
            100                 105                 110

Ala Val Gln Met Ser Thr Lys Val Leu Ile Gln Phe Leu Gln Lys Lys
        115                 120                 125

Ala Lys Asn Leu Asp Ala Ile Thr Thr Pro Asp Pro Thr Thr Asn Ala
    130                 135                 140

Ser Leu Leu Thr Lys Leu Gln Ala Gln Asn Gln Trp Leu Gln Asp Met
145                 150                 155                 160

Thr Thr His Leu Ile Leu Arg Ser Phe Lys Glu Phe Leu Gln Ser Ser
                165                 170                 175

Leu Arg Ala Leu Arg Gln Met Glu Pro Lys Ser Ser Asp Lys Thr His
            180                 185                 190

Thr Cys Pro Pro Cys Pro Ala Pro Glu Leu Leu Gly Gly Pro Ser Val
        195                 200                 205

Phe Leu Phe Pro Pro Lys Pro Lys Asp Thr Leu Met Ile Ser Arg Thr
    210                 215                 220

Pro Glu Val Thr Cys Val Val Val Asp Val Ser His Glu Asp Pro Glu
225                 230                 235                 240

Val Lys Phe Asn Trp Tyr Val Asp Gly Val Glu Val His Asn Ala Lys
                245                 250                 255

Thr Lys Pro Arg Glu Glu Gln Tyr Asn Ser Thr Tyr Arg Val Val Ser
            260                 265                 270

Val Leu Thr Val Leu His Gln Asp Trp Leu Asn Gly Lys Glu Tyr Lys
        275                 280                 285

Cys Lys Val Ser Asn Lys Ala Leu Pro Ala Pro Ile Glu Lys Thr Ile
    290                 295                 300

Ser Lys Ala Lys Gly Gln Pro Arg Glu Pro Gln Val Tyr Thr Leu Pro
305                 310                 315                 320

Pro Ser Arg Asp Glu Leu Thr Lys Asn Gln Val Ser Leu Thr Cys Leu
                325                 330                 335

Val Lys Gly Phe Tyr Pro Ser Asp Ile Ala Val Glu Trp Glu Ser Asn
            340                 345                 350

Gly Gln Pro Glu Asn Asn Tyr Lys Thr Thr Pro Pro Val Leu Asp Ser
        355                 360                 365

Asp Gly Ser Phe Phe Leu Tyr Ser Lys Leu Thr Val Asp Lys Ser Arg

370                 375                 380

Trp Gln Gln Gly Asn Val Phe Ser Cys Ser Val Met His Glu Ala Leu
385                 390                 395                 400

His Asn His Tyr Thr Gln Lys Ser Leu Ser Leu Ser Pro Gly Lys
                405                 410                 415

<210>  6
<211>  189
<212>  PRT
<213>  artificial sequence

<220>
<223>  IL-6.his

<400>  6

Val Pro Pro Gly Glu Asp Ser Lys Asp Val Ala Ala Pro His Arg Gln
1               5                   10                  15

Pro Leu Thr Ser Ser Glu Arg Ile Asp Lys Gln Ile Arg Tyr Ile Leu
            20                  25                  30

Asp Gly Ile Ser Ala Leu Arg Lys Glu Thr Cys Asn Lys Ser Asn Met
        35                  40                  45

Cys Glu Ser Ser Lys Glu Ala Leu Ala Glu Asn Asn Leu Asn Leu Pro
    50                  55                  60

Lys Met Ala Glu Lys Asp Gly Cys Phe Gln Ser Gly Phe Asn Glu Glu
65                  70                  75                  80

Thr Cys Leu Val Lys Ile Ile Thr Gly Leu Leu Glu Phe Glu Val Tyr
                85                  90                  95

Leu Glu Tyr Leu Gln Asn Arg Phe Glu Ser Ser Glu Glu Gln Ala Arg
            100                 105                 110

Ala Val Gln Met Ser Thr Lys Val Leu Ile Gln Phe Leu Gln Lys Lys
        115                 120                 125

Ala Lys Asn Leu Asp Ala Ile Thr Thr Pro Asp Pro Thr Thr Asn Ala
    130                 135                 140

Ser Leu Leu Thr Lys Leu Gln Ala Gln Asn Gln Trp Leu Gln Asp Met

145                 150                 155                 160

Thr Thr His Leu Ile Leu Arg Ser Phe Lys Glu Phe Leu Gln Ser Ser
                165                 170                 175

Leu Arg Ala Leu Arg Gln Met His His His His His His
            180                 185

<210>  7
<211>  829
<212>  PRT
<213>  artificial sequence

<220>
<223>  gp130.Fc

<400>  7

Glu Leu Leu Asp Pro Cys Gly Tyr Ile Ser Pro Glu Ser Pro Val Val
1               5                   10                  15

Gln Leu His Ser Asn Phe Thr Ala Val Cys Val Leu Lys Glu Lys Cys
            20                  25                  30

Met Asp Tyr Phe His Val Asn Ala Asn Tyr Ile Val Trp Lys Thr Asn
        35                  40                  45

His Phe Thr Ile Pro Lys Glu Gln Tyr Thr Ile Ile Asn Arg Thr Ala
    50                  55                  60

Ser Ser Val Thr Phe Thr Asp Ile Ala Ser Leu Asn Ile Gln Leu Thr
65                  70                  75                  80

Cys Asn Ile Leu Thr Phe Gly Gln Leu Glu Gln Asn Val Tyr Gly Ile
                85                  90                  95

Thr Ile Ile Ser Gly Leu Pro Pro Glu Lys Pro Lys Asn Leu Ser Cys
            100                 105                 110

Ile Val Asn Glu Gly Lys Lys Met Arg Cys Glu Trp Asp Gly Gly Arg
        115                 120                 125

Glu Thr His Leu Glu Thr Asn Phe Thr Leu Lys Ser Glu Trp Ala Thr
    130                 135                 140

His Lys Phe Ala Asp Cys Lys Ala Lys Arg Asp Thr Pro Thr Ser Cys

145                 150                 155                 160

Thr Val Asp Tyr Ser Thr Val Tyr Phe Val Asn Ile Glu Val Trp Val
                165                 170                 175

Glu Ala Glu Asn Ala Leu Gly Lys Val Thr Ser Asp His Ile Asn Phe
            180                 185                 190

Asp Pro Val Tyr Lys Val Lys Pro Asn Pro Pro His Asn Leu Ser Val
        195                 200                 205

Ile Asn Ser Glu Glu Leu Ser Ser Ile Leu Lys Leu Thr Trp Thr Asn
    210                 215                 220

Pro Ser Ile Lys Ser Val Ile Ile Leu Lys Tyr Asn Ile Gln Tyr Arg
225                 230                 235                 240

Thr Lys Asp Ala Ser Thr Trp Ser Gln Ile Pro Pro Glu Asp Thr Ala
                245                 250                 255

Ser Thr Arg Ser Ser Phe Thr Val Gln Asp Leu Lys Pro Phe Thr Glu
            260                 265                 270

Tyr Val Phe Arg Ile Arg Cys Met Lys Glu Asp Gly Lys Gly Tyr Trp
        275                 280                 285

Ser Asp Trp Ser Glu Glu Ala Ser Gly Ile Thr Tyr Glu Asp Arg Pro
    290                 295                 300

Ser Lys Ala Pro Ser Phe Trp Tyr Lys Ile Asp Pro Ser His Thr Gln
305                 310                 315                 320

Gly Tyr Arg Thr Val Gln Leu Val Trp Lys Thr Leu Pro Pro Phe Glu
                325                 330                 335

Ala Asn Gly Lys Ile Leu Asp Tyr Glu Val Thr Leu Thr Arg Trp Lys
            340                 345                 350

Ser His Leu Gln Asn Tyr Thr Val Asn Ala Thr Lys Leu Thr Val Asn
        355                 360                 365

Leu Thr Asn Asp Arg Tyr Leu Ala Thr Leu Thr Val Arg Asn Leu Val
    370                 375                 380

Gly Lys Ser Asp Ala Ala Val Leu Thr Ile Pro Ala Cys Asp Phe Gln
385                 390                 395                 400

Ala Thr His Pro Val Met Asp Leu Lys Ala Phe Pro Lys Asp Asn Met
                405                 410                 415

Leu Trp Val Glu Trp Thr Thr Pro Arg Glu Ser Val Lys Lys Tyr Ile
            420                 425                 430

Leu Glu Trp Cys Val Leu Ser Asp Lys Ala Pro Cys Ile Thr Asp Trp
        435                 440                 445

Gln Gln Glu Asp Gly Thr Val His Arg Thr Tyr Leu Arg Gly Asn Leu
    450                 455                 460

Ala Glu Ser Lys Cys Tyr Leu Ile Thr Val Thr Pro Val Tyr Ala Asp
465                 470                 475                 480

Gly Pro Gly Ser Pro Glu Ser Ile Lys Ala Tyr Leu Lys Gln Ala Pro
                485                 490                 495

Pro Ser Lys Gly Pro Thr Val Arg Thr Lys Lys Val Gly Lys Asn Glu
            500                 505                 510

Ala Val Leu Glu Trp Asp Gln Leu Pro Val Asp Val Gln Asn Gly Phe
        515                 520                 525

Ile Arg Asn Tyr Thr Ile Phe Tyr Arg Thr Ile Ile Gly Asn Glu Thr
    530                 535                 540

Ala Val Asn Val Asp Ser Ser His Thr Glu Tyr Thr Leu Ser Ser Leu
545                 550                 555                 560

Thr Ser Asp Thr Leu Tyr Met Val Arg Met Ala Ala Tyr Thr Asp Glu
                565                 570                 575

Gly Gly Lys Asp Gly Pro Glu Phe Thr Phe Thr Thr Pro Lys Phe Ala
            580                 585                 590

Gln Gly Glu Ile Glu Glu Pro Lys Ser Ser Asp Lys Thr His Thr Cys
        595                 600                 605

Pro Pro Cys Pro Ala Pro Glu Leu Leu Gly Gly Pro Ser Val Phe Leu

610                 615                 620

Phe Pro Pro Lys Pro Lys Asp Thr Leu Met Ile Ser Arg Thr Pro Glu
625                 630                 635                 640

Val Thr Cys Val Val Val Asp Val Ser His Glu Asp Pro Glu Val Lys
                645                 650                 655

Phe Asn Trp Tyr Val Asp Gly Val Glu Val His Asn Ala Lys Thr Lys
            660                 665                 670

Pro Arg Glu Glu Gln Tyr Asn Ser Thr Tyr Arg Val Val Ser Val Leu
        675                 680                 685

Thr Val Leu His Gln Asp Trp Leu Asn Gly Lys Glu Tyr Lys Cys Lys
    690                 695                 700

Val Ser Asn Lys Ala Leu Pro Ala Pro Ile Glu Lys Thr Ile Ser Lys
705                 710                 715                 720

Ala Lys Gly Gln Pro Arg Glu Pro Gln Val Tyr Thr Leu Pro Pro Ser
                725                 730                 735

Arg Asp Glu Leu Thr Lys Asn Gln Val Ser Leu Thr Cys Leu Val Lys
            740                 745                 750

Gly Phe Tyr Pro Ser Asp Ile Ala Val Glu Trp Glu Ser Asn Gly Gln
        755                 760                 765

Pro Glu Asn Asn Tyr Lys Thr Thr Pro Pro Val Leu Asp Ser Asp Gly
    770                 775                 780

Ser Phe Phe Leu Tyr Ser Lys Leu Thr Val Asp Lys Ser Arg Trp Gln
785                 790                 795                 800

Gln Gly Asn Val Phe Ser Cys Ser Val Met His Glu Ala Leu His Asn
                805                 810                 815

His Tyr Thr Gln Lys Ser Leu Ser Leu Ser Pro Gly Lys
            820                 825

<210>  8
<211>  449
<212>  PRT

<213>  artificial sequence

<220>
<223>  Tocilizumab heavy chain

<400>  8

Glu Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly

435                 440                 445

Lys

<210>  9
<211>  214
<212>  PRT
<213>  artificial sequence

<220>
<223>  Tocilizumab (Toci) light chain

<400>  9

<210>  10
<211>  353
<212>  PRT
<213>  artificial sequence

<220>
<223>  20A11-Fc fusion protein (20A11)

<400>  10

Glu Val Gln Leu Val Glu Ser Gly Gly Gly Leu Val Gln Pro Gly Gly
1               5                   10                  15

Ser Leu Arg Leu Ser Cys Ala Ala Ser Gly Ser Val Phe Lys Ile Asn
            20                  25                  30

Val Met Ala Trp Tyr Arg Gln Ala Pro Gly Lys Gly Arg Glu Leu Val
        35                  40                  45

Ala Gly Ile Ile Ser Gly Gly Ser Thr Ser Tyr Ala Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Ser Leu Arg Pro Glu Asp Thr Ala Val Tyr Tyr Cys Ala
                85                  90                  95

Phe Ile Thr Thr Glu Ser Asp Tyr Asp Leu Gly Arg Arg Tyr Trp Gly
            100                 105                 110

Gln Gly Thr Leu Val Thr Val Ser Ser Glu Pro Lys Ser Ser Asp Lys
        115                 120                 125

Thr His Thr Cys Pro Pro Cys Pro Ala Pro Glu Leu Leu Gly Gly Pro

    130                 135                 140

Ser Val Phe Leu Phe Pro Pro Lys Pro Lys Asp Thr Leu Met Ile Ser
145                 150                 155                 160

Arg Thr Pro Glu Val Thr Cys Val Val Val Asp Val Ser His Glu Asp
                165                 170                 175

Pro Glu Val Lys Phe Asn Trp Tyr Val Asp Gly Val Glu Val His Asn
            180                 185                 190

Ala Lys Thr Lys Pro Arg Glu Glu Gln Tyr Asn Ser Thr Tyr Arg Val
        195                 200                 205

Val Ser Val Leu Thr Val Leu His Gln Asp Trp Leu Asn Gly Lys Glu
    210                 215                 220

Tyr Lys Cys Lys Val Ser Asn Lys Ala Leu Pro Ala Pro Ile Glu Lys
225                 230                 235                 240

Thr Ile Ser Lys Ala Lys Gly Gln Pro Arg Glu Pro Gln Val Tyr Thr
                245                 250                 255

Leu Pro Pro Ser Arg Asp Glu Leu Thr Lys Asn Gln Val Ser Leu Thr
            260                 265                 270

Cys Leu Val Lys Gly Phe Tyr Pro Ser Asp Ile Ala Val Glu Trp Glu
        275                 280                 285

Ser Asn Gly Gln Pro Glu Asn Asn Tyr Lys Thr Thr Pro Pro Val Leu
    290                 295                 300

Asp Ser Asp Gly Ser Phe Phe Leu Tyr Ser Lys Leu Thr Val Asp Lys
305                 310                 315                 320

Ser Arg Trp Gln Gln Gly Asn Val Phe Ser Cys Ser Val Met His Glu
                325                 330                 335

Ala Leu His Asn His Tyr Thr Gln Lys Ser Leu Ser Leu Ser Pro Gly
            340                 345                 350

Lys

<210>  11
<211>  446
<212>  PRT
<213>  artificial sequence

<220>
<223>  Sarilumab (Sari) heavy chain

<400>  11

Glu Val Gln Leu Val Glu Ser Gly Gly Gly Leu Val Gln Pro Gly Arg
1               5                   10                  15

Ser Leu Arg Leu Ser Cys Ala Ala Ser Arg Phe Thr Phe Asp Asp Tyr
            20                  25                  30

Ala Met His Trp Val Arg Gln Ala Pro Gly Lys Gly Leu Glu Trp Val
        35                  40                  45

Ser Gly Ile Ser Trp Asn Ser Gly Arg Ile Gly Tyr Ala Asp Ser Val
    50                  55                  60

Lys Gly Arg Phe Thr Ile Ser Arg Asp Asn Ala Glu Asn Ser Leu Phe
65                  70                  75                  80

Leu Gln Met Asn Gly Leu Arg Ala Glu Asp Thr Ala Leu Tyr Tyr Cys
                85                  90                  95

Ala Lys Gly Arg Asp Ser Phe Asp Ile Trp Gly Gln Gly Thr Met Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Asp Glu Leu Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His

420                 425                 430

Asn His Tyr Thr Gln Lys Ser Leu Ser Leu Ser Pro Gly Lys
        435                 440                 445

<210>  12
<211>  214
<212>  PRT
<213>  artificial sequence

<220>
<223>  Sarilumab (Sari) light chain

<400>  12

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Gly Lys Ala Pro Lys Leu Leu Ile
        35                  40                  45

Tyr Gly Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Ser Tyr Tyr Cys Gln Gln Ala Asn Ser Phe Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser

165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  13
<211>  363
<212>  DNA
<213>  artificial sequence

<220>
<223>  1764 VHH coding nucleic acid

<400>  13
gaggtgcagc tggtggagtc tgggggaggg ttggtgcagg ctggggggtc tctgagactc      60

tcctgtgaag cctctggaaa catcttcaag atcaatgtca tgggctggta ccgccaggct     120

ccagggaagc agcgcgagtg ggtcgcagct attattagtg gcggtagcac aaactatgca     180

gactccgtga agggccgatt caccatctcc agagacaacg ccaagaacac ggtgtatctg     240

caaatgaaca gcctgaaacc tgaggacacg gccgtctatt actgtaatgc tattctcacc     300

tataacgact atgacctagg gtctgactac tggggccagg ggacccaggt caccgtctcc     360

tca                                                                   363

<210>  14
<211>  121
<212>  PRT
<213>  artificial sequence

<220>
<223>  1764 VHH

<400>  14

Glu Val Gln Leu Val Glu Ser Gly Gly Gly Leu Val Gln Ala Gly Gly
1               5                   10                  15

Ser Leu Arg Leu Ser Cys Glu Ala Ser Gly Asn Ile Phe Lys Ile Asn
            20                  25                  30

Val Met Gly Trp Tyr Arg Gln Ala Pro Gly Lys Gln Arg Glu Trp Val

35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Val Tyr Tyr Cys Asn
                85                  90                  95

Ala Ile Leu Thr Tyr Asn Asp Tyr Asp Leu Gly Ser Asp Tyr Trp Gly
            100                 105                 110

Gln Gly Thr Gln Val Thr Val Ser Ser
        115                 120

<210>  15
<211>  5
<212>  PRT
<213>  artificial sequence

<220>
<223>  1764 CDR1

<400>  15

Ile Asn Val Met Gly
1               5

<210>  16
<211>  16
<212>  PRT
<213>  artificial sequence

<220>
<223>  1764 CDR2

<400>  16

Ala Ile Ile Ser Gly Gly Ser Thr Asn Tyr Ala Asp Ser Val Lys Gly
1               5                   10                  15

<210>  17
<211>  13
<212>  PRT
<213>  artificial sequence

<220>
<223>  1764 CDR3

<400>  17

Ile Leu Thr Tyr Asn Asp Tyr Asp Leu Gly Ser Asp Tyr
1               5                   10

<210>  18
<211>  699
<212>  DNA
<213>  artificial sequence

<220>
<223>  human Fc fragment

<400>  18
gagcccaaat ctagtgacaa aactcacacg tgcccaccgt gcccagcacc tgaactcctg      60

gggggaccgt cagtcttcct cttcccccca aaacccaagg acaccctcat gatctcccgg     120

acccctgagg tcacatgcgt ggtggtggac gtgagccacg aagaccctga ggtcaagttc     180

aactggtacg tggacggcgt ggaggtgcat aatgccaaga caaagccgcg ggaagagcag     240

tacaacagca cgtaccgtgt ggtcagcgtc ctcaccgtcc tgcaccagga ctggctgaat     300

ggcaaggagt acaagtgcaa ggtctccaac aaagccctcc cagcccccat cgagaaaacc     360

atctccaaag ccaaagggca gccccgagaa ccacaggtgt acaccctgcc cccatcccgg     420

gatgagctga ccaagaacca ggtcagcctg acctgcctgg tcaaaggctt ctatcccagc     480

gacatcgccg tggagtggga gagcaatggg cagccggaga acaactacaa gaccacgcct     540

cccgtgctgg actccgacgg ctccttcttc ctctacagca agctcaccgt ggacaagagc     600

aggtggcagc aggggaacgt cttctcatgc tccgtgatgc atgaggctct gcacaaccac     660

tacacgcaga agagcctctc cctgtctccg ggtaaatga                            699

<210>  19
<211>  232
<212>  PRT
<213>  artificial sequence

<220>
<223>  human Fc fragment

<400>  19

Glu Pro Lys Ser Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210> 20
<211> 121
<212> PRT
<213> artificial sequence

<220>
<223>  1764-mu1

<400>  20

Glu Val Gln Leu Val Glu Ser Gly Gly Gly Leu Val Gln Pro Gly Gly
1               5                   10                  15

Ser Leu Arg Leu Ser Cys Glu Ala Ser Gly Asn Ile Phe Lys Ile Asn
            20                  25                  30

Val Met Gly Trp Tyr Arg Gln Ala Pro Gly Lys Gln Arg Glu Trp Val
        35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Arg Pro Glu Asp Thr Ala Val Tyr Tyr Cys Asn
                85                  90                  95

Ala Ile Leu Thr Tyr Asn Asp Tyr Asp Leu Gly Ser Asp Tyr Trp Gly
            100                 105                 110

Gln Gly Thr Gln Val Thr Val Ser Ser
        115                 120


<210>  21
<211>  121
<212>  PRT
<213>  artificial sequence

<220>
<223>  1764-mu2

<400>  21

Glu Val Gln Leu Val Glu Ser Gly Gly Gly Leu Val Gln Ala Gly Gly
1               5                   10                  15

Ser Leu Arg Leu Ser Cys Ala Ala Ser Gly Asn Ile Phe Lys Ile Asn
            20                  25                  30

Val Met Gly Trp Tyr Arg Gln Ala Pro Gly Lys Gly Arg Glu Trp Val
        35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Val Tyr Tyr Cys Asn
                85                  90                  95

Ala Ile Leu Thr Tyr Asn Asp Tyr Asp Leu Gly Ser Asp Tyr Trp Gly
            100                 105                 110

Gln Gly Thr Gln Val Thr Val Ser Ser
        115                 120

<210>  22
<211>  121
<212>  PRT
<213>  artificial sequence

<220>
<223>  1764-mu3

<400>  22

Glu Val Gln Leu Val Glu Ser Gly Gly Gly Leu Val Gln Pro Gly Gly
1               5                   10                  15

Ser Leu Arg Leu Ser Cys Glu Ala Ser Gly Asn Ile Phe Lys Ile Asn
            20                  25                  30

Val Met Gly Trp Tyr Arg Gln Ala Pro Gly Lys Gln Arg Glu Trp Val
        35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Arg Pro Glu Asp Thr Ala Val Tyr Tyr Cys Asn
                85                  90                  95

Ala Ile Leu Thr Tyr Asn Asp Tyr Asp Leu Gly Ser Asp Tyr Trp Gly
            100                 105                 110

Gln Gly Thr Leu Val Thr Val Ser Ser
        115                 120

<210>  23
<211>  121
<212>  PRT
<213>  artificial sequence

<220>
<223>  1764-mu4

<400>  23

Glu Val Gln Leu Val Glu Ser Gly Gly Gly Leu Val Gln Ala Gly Gly
1               5                   10                  15

Ser Leu Arg Leu Ser Cys Ala Ala Ser Gly Asn Ile Phe Lys Ile Asn
            20                  25                  30

Val Met Gly Trp Tyr Arg Gln Ala Pro Gly Lys Gly Arg Glu Trp Val
        35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Val Tyr Tyr Cys Asn
                85                  90                  95

Ala Ile Leu Thr Tyr Asn Asp Tyr Asp Leu Gly Ser Asp Tyr Trp Gly
            100                 105                 110

Gln Gly Thr Leu Val Thr Val Ser Ser
        115                 120

<210>  24
<211>  121
<212>  PRT
<213>  artificial sequence

<220>
<223>  1764-mu5

<400>  24

Glu Val Gln Leu Val Glu Ser Gly Gly Gly Leu Val Gln Ala Gly Gly

1               5                   10                  15

Ser Leu Arg Leu Ser Cys Ala Ala Ser Gly Asn Ile Phe Lys Ile Asn
            20                  25                  30

Val Met Gly Trp Tyr Arg Gln Ala Pro Gly Lys Gln Arg Glu Trp Val
        35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Val Tyr Tyr Cys Ala
                85                  90                  95

Phe Ile Leu Thr Tyr Asn Asp Tyr Asp Leu Gly Ser Asp Tyr Trp Gly
            100                 105                 110

Gln Gly Thr Leu Val Thr Val Ser Ser
        115                 120

<210>  25
<211>  121
<212>  PRT
<213>  artificial sequence

<220>
<223>  1764-mu6

<400>  25

Glu Val Gln Leu Val Glu Ser Gly Gly Gly Leu Val Gln Pro Gly Gly
1               5                   10                  15

Ser Leu Arg Leu Ser Cys Ala Ala Ser Gly Asn Ile Phe Lys Ile Asn
            20                  25                  30

Val Met Gly Trp Tyr Arg Gln Ala Pro Gly Lys Gly Arg Glu Trp Val
        35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Leu Tyr Leu

65                  70                  75                  80

Gln Met Asn Ser Leu Arg Pro Glu Asp Thr Ala Val Tyr Tyr Cys Asn
                85                  90                  95

Ala Ile Leu Thr Tyr Asn Asp Tyr Asp Leu Gly Ser Asp Tyr Trp Gly
            100                 105                 110

Gln Gly Thr Leu Val Thr Val Ser Ser
        115                 120

<210>  26
<211>  121
<212>  PRT
<213>  artificial sequence

<220>
<223>  1764-mu7

<400>  26

Glu Val Gln Leu Val Glu Ser Gly Gly Gly Leu Val Gln Pro Gly Gly
1               5                   10                  15

Ser Leu Arg Leu Ser Cys Ala Ala Ser Gly Asn Ile Phe Lys Ile Asn
            20                  25                  30

Val Met Gly Trp Tyr Arg Gln Ala Pro Gly Lys Gln Arg Glu Trp Val
        35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Ser Leu Arg Pro Glu Asp Thr Ala Val Tyr Tyr Cys Ala
                85                  90                  95

Phe Ile Leu Thr Tyr Asn Asp Tyr Asp Leu Gly Ser Asp Tyr Trp Gly
            100                 105                 110

Gln Gly Thr Leu Val Thr Val Ser Ser
        115                 120

<210>  27

<211>  121
<212>  PRT
<213>  artificial sequence

<220>
<223>  1764-mu8

<400>  27

Glu Val Gln Leu Val Glu Ser Gly Gly Gly Leu Val Gln Ala Gly Gly
1               5                   10                  15

Ser Leu Arg Leu Ser Cys Ala Ala Ser Gly Asn Ile Phe Lys Ile Asn
            20                  25                  30

Val Met Gly Trp Tyr Arg Gln Ala Pro Gly Lys Gly Arg Glu Trp Val
        35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Ser Leu Lys Pro Glu Asp Thr Ala Val Tyr Tyr Cys Ala
                85                  90                  95

Phe Ile Leu Thr Tyr Asn Asp Tyr Asp Leu Gly Ser Asp Tyr Trp Gly
            100                 105                 110

Gln Gly Thr Leu Val Thr Val Ser Ser
        115                 120


<210>  28
<211>  126
<212>  PRT
<213>  artificial sequence

<220>
<223>  1764-mu9

<400>  28

Glu Val Gln Leu Val Glu Ser Gly Gly Gly Leu Val Gln Pro Gly Gly
1               5                   10                  15

Ser Leu Arg Leu Ser Cys Ala Ala Ser Gly Asn Ile Phe Lys Ile Asn
            20                  25                  30

Val Met Gly Trp Tyr Arg Gln Ala Pro Gly Lys Gly Arg Glu Trp Val
        35                  40                  45

Ala Ala Ile Ile Ser Gly Gly Ser Thr Asn Tyr Ala Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Ser Leu Arg Pro Glu Asp Thr Ala Val Tyr Tyr Cys Ala
                85                  90                  95

Phe Ile Leu Thr Tyr Asn Asp Tyr Asp Leu Gly Ser Asp Tyr Trp Gly
            100                 105                 110

Gln Gly Thr Leu Val Thr Val Ser Ser Glu Pro Lys Ser Ser
        115                 120                 125